# EUROPEAN PATENT SPECIFICATION

(11) **EP 4 132 405 B1**
(45) Date of publication and mention of the grant of the patent: **07.05.2025**
(21) Application number: 21714910.3
(22) Date of filing: 31.03.2021
(51) Int. Cl.: A61B 34/10, A61B 17/34, A61N 5/10, G16H 20/40, A61B 34/00, A61B 34/20, A61B 90/00, A61B 90/11

(54) **GRID TEMPLATE POSITIONING IN INTERVENTIONAL MEDICINE**
RASTERSCHABLONENPOSITIONIERUNG IN DER INTERVENTIONELLEN MEDIZIN
POSITIONNEMENT DE MODÈLE DE GRILLE EN MÉDECINE INTERVENTIONNELLE

(30) Priority: 10.04.2020 EP 20169170
(43) Date of publication of application: 15.02.2023
(73) Proprietor: Koninklijke Philips N.V., 5656 AG Eindhoven (NL)
(72) Inventor: ISOLA, Alfonso Agatino, 5656 AE Eindhoven (NL); HAUTVAST, Guillaume Leopold Theodorus Frederik, 5656 AE Eindhoven (NL)
(74) Representative: Philips Intellectual Property & Standards
(86) International application number: PCT/EP2021/058430
(87) International publication number: WO 2021/204622

(56) References cited:
- WO-A1-2016/007717
- WO-A1-2016/059603
- WO-A1-2018/108952

## Description

### FIELD OF THE INVENTION

The present invention relates to the field of planning of interventional procedures, such as brachytherapy and ablation procedures. Specifically, the invention relates to devices and methods for the planning of a procedure in which a grid, or template, is used for supporting and guiding interventional tools to be inserted through selected holes of the grid into an anatomical volume of interest, such as needles, catheters and the like.

### BACKGROUND OF THE INVENTION

In focal treatment procedures, one or more focal sources are used at close proximity to and/or inside a treatment volume in the body, such as a cancer lesion. Such focal sources may be integrated in, or positioned by, interventional focal therapy delivery devices, such as needles or catheters. Focal sources can provide a highly localized energy exposure in order to damage cells in the treatment volume, while safeguarding healthy tissues outside this volume. Generally, and without necessarily being strictly limiting thereto, focal sources may deposit energy in accordance with an inverse square law to achieve this advantageous highly focused (localized) treatment effect. Many modalities of focal therapy exist in the art. Possibly the best known modality is brachytherapy, in which one or more radioactive seeds (sealed radioisotopes) are placed inside or close to the area requiring treatment. Other examples include thermal ablation, microwave ablation, ultrasound ablation, radiofrequency ablation, photothermal therapy, laser therapy, and the like. Specific sensitizing techniques can also be used, such as in photodynamic therapy. While, in a strict sense, cryoablation does not rely on a focal energy source, but instead uses a focal heat sink, it can be considered as entirely analogous, and therefore as yet another example of a focal therapy modality.

It is known in the art to use a rigid or flexible grid template, positioned on the body of the patient, to guide and support the focal therapy delivery device(s), such as needles.

For example, in prostate brachytherapy, needles or catheters may be inserted into the prostate and/or surrounding tissue through such an alignment template that is positioned against the perineum. The template thus guides, supports and (temporarily) secures the needles or catheters such that the intended delivery site(s) in the body is reached with a good positional accuracy and such that the treatment tool(s) remain at their intended site for a predetermined required time during the procedure.

While the present disclosure aims to provide means and methods for planning and executing an accurate positioning of such grid template relative to the body and predetermined volumes therein, which is particularly useful for focal therapies, embodiments are not necessarily limited thereto. Other interventional procedures in which a grid template to insert a tool into a precise location in a tissue of the body can be used may also benefit from the present invention, such as biopsies.

Typically, a medical imaging technique may be used to identify and segment regions of interest in the body, such as the primary treatment volume and possibly nearby organs at risk. This information can then be used to perform a manual forward planning or an automatic inverse planning to find a set of delivery parameters, which may include, for example, a selection of catheter or needle positions on the grid template, insertion depths therefor, dwell (or ablation) times and/or treatment power parameters (e.g. radioisotope flux or ablation power). For example, in brachytherapy inverse planning methods, the grid hole trajectories that intersect with the lesion regions to be treated may define target lesion intersection segments used to sample the set of dwell positions at which the radioactive source could dwell for a given optimal time. In a typical planning, a plurality of such available trajectories may be selected, and used in a sequential treatment pattern, e.g. a brachytherapy radiation source (or an ablation tool) is inserted through each selected hole in accordance with the determined parameters, e.g. in accordance with determined dwell times and insertion depths.

However, this planning is constrained by the positioning of the grid template, which typically comprises a plurality of through-holes (e.g. arranged in a grid pattern) that define the available positions of the delivery devices on this predefined grid, e.g. along paths through the holes and perpendicular to the grid template.

For example, e.g. for particularly small lesions, a suboptimal grid position could limit the maximum quality of treatment plan that can be achieved under the planning constraints. In a worst-case scenario, a very small lesion could be positioned entirely between grid hole trajectories. The optimal treatment within these constraints could therefore increase the number of focal therapy sources, thereby surrounding the lesion as much as possible, with an undesirable increase of damage to surrounding, presumably healthy, tissue, e.g. increasing the risk of unwanted side effects. Unfortunately, such scenario is not uncommon in clinical practice. For example, a clinical expert may include a margin, e.g. 1-2 centimeters, around the gross tumor volume to enlarge the target volume and to obtain some intersecting grid trajectories. In such event, no trajectories, and therefore also no suitable brachytherapy source or ablator dwell positions, would be available for optimization without taking the additional margin into account. However, this additional margin also implies that healthy tissues near the gross tumour may be affected, e.g. destroyed by the therapy, which may lead to potential side effects.

However, this approach also has its advantages. By limiting the possible needle positions to a predetermined grid, the complexity of the planning algorithm is reduced, e.g. can remain tractable. For example, since the position of the needles is constrained to a possibly large, yet finite (discrete) number, direct search and/or integer programming planning strategies can be used. Likewise, due to the finite size of the brachytherapy seed or ablation tool, the number of dwell positions (i.e. having a substantially distinct effect) can also be constrained to a discrete number. While robotic systems are known in the art that allow a substantially continuous positioning of an interventional tool with respect to a predefined coordinate system, a template-based approach still offers an advantageously cost-effective, simple and quick solution, which in many scenarios may be preferable.

A document WO 2016/059603 A1 discloses an interventional therapy system including at least one catheter configured for insertion within an object of interest; and at least one controller which: obtains a reference image dataset comprising a plurality of image slices which form a three-dimensional image of the object of interest, defines restricted areas within the reference image dataset, determines location constraints for the at least one catheter in accordance with at least one of planned catheter intersection points, a peripheral boundary of the object of interest and the restricted areas defined in the reference dataset, determines at least one of a position and an orientation of the distal end of the at least one catheter, and/or determines a planned trajectory for the at least one catheter in accordance with the determined at least one position and orientation for the at least one catheter and the location constraints.

EP1374949 describes an illustrative approach to brachytherapy planning. This reference discloses a real time radiation treatment planning system. A three-dimensional image segmentation algorithm is used to determine specific organs in an anatomical region of interest. A treatment plan for effecting the radiation therapy is then determined that defines the number and position of hollow needles in the anatomical region and the radiation dose to be delivered. A single or multi-objective anatomy-based genetic optimization algorithm is used to determine in real time an optimal number and position of the hollow needle(s), a position of energy emitting source in each hollow needle and the dwell times of the energy emitting source at each position. The needle(s) is then inserted under the guidance of a template or guidance tool into the anatomical region and an energy emitting source is delivered through the hollow needle. Furthermore, the achieved needle positions and dwell times can be determined, for post-planning purposes, based on three-dimensional image information. A specific embodiment described in this disclosure relates to a motorized template without holes, which uses a single guiding tube through which the needle can be inserted. This guiding tube can be positioned in each position of a virtual template grid, such that the positioning of the needle in relation to the template is not limited by a physical grid of holes. Thus, the virtual grid configuration is only limited to the diameter of the needles used.

### SUMMARY OF THE INVENTION

It is an object of embodiments of the present invention to provide easy, effective, efficient and/or good means and/or methods for determining a position and/or orientation for a grid template, e.g. on or near the surface of the body of a patient, for supporting and guiding interventional tools to be inserted through holes of the grid into an anatomical volume of interest, such as needles, catheters and the like, in an interventional procedure, such as a brachytherapy or ablation procedure.

A device and method in accordance with embodiments of the present invention achieves the above objective.

It is an advantage of embodiments of the present invention that the position of the grid is optimized to provide a good accessibility (e.g. a good physical access and/or coverage) of a treatment volume of interest in the body by delivery paths (for needles, catheters or other interventional tools) through grid holes of the grid when placed in its determined position.

It is an advantage of embodiments of the present invention that the delivery paths through the grid holes can be optimized to intersect as much as possible with the treatment volume of interest.

It is an advantage of embodiments of the present invention that a good treatment efficacy of procedures, such as ablation or brachytherapy, can be achieved, e.g. in the treatment of non-resectable tumors. It is a further advantage that undesirable side-effects can be reduced or avoided, e.g. that damage to healthy tissues can be avoided or reduced.

It is a further advantage that a treatment dose (e.g. radiation dose, ablation energy) can be reduced by enabling a precise delivery of the dose in the treatment volume. It is a further advantage that a good recovery (e.g. rapid, e.g. without undesirable side-effects) of the patient after treatment can be achieved. For example, to reduce unwanted damage to nearby healthy tissues, the total delivered radioactive dose or thermal ablation zone should conform as much as possible to the tumor contours (possibly including a margin around the tumor as deemed suitable by the clinician, and affected as little as possible by constraints imposed by the grid).

It is an advantage of embodiments of the present invention that the presently disclosed approach can be applied to a wide variety of interventional procedures in which the precise positioning of an interventional tool(s) in a volume of interest inside the body, via delivery paths defined by a template grid, is advantageous. For example, the present approach may be applied without requiring knowledge of the specific procedure to be applied, e.g. only taking into account an aim of providing good access over a predetermined volume of interest in the body via delivery paths provided by the template grid, preferably in as many positions sampled across the volume as possible, and preferably with a distribution of these positions over the volume as uniform as possible. It is an advantage that further constraints can be taken into account, such as regions at risk to be avoided; such as tissue that could be damaged by physical insertion of an interventional tool into and/or through the region at risk and/or by an energy source, such as an ablation tool or brachytherapy seed, when positioned in or near the region at risk.

It is an advantage of embodiments of the present invention that the present approach can be used for readily available, cheap, disposable and/or simple template grids, e.g. as known in the art.

It is an advantage of embodiments of the present invention that the use of simple template grids may aid in avoiding compatibility problems with more complex positioning aids, such as compatibility with magnetic resonance imaging, echography and/or x-ray imaging.

It is an advantage of embodiments of the present invention that complex robotic systems for a substantially free positioning of an interventional tool can be avoided, e.g. robotic systems for substantially continuous positioning of an interventional tool in three (four, five or six) degrees of (roto)translational freedom, while still providing many benefits of such complex systems in terms of accuracy. For example, while a robotic system, with substantial positioning freedom, may allow a good optimization of the treatment, it may come at a heavy cost, e.g. in terms of initial investment, maintenance requirements and training of clinical staff. It is an advantage of embodiments that cheap, simple and readily available treatment systems, e.g. grid templates and/or treatment planning systems, can be used, while still achieving a high-quality treatment, e.g. allowing a better optimization than conventionally achieved by treatments relying on a manually positioned grid template.

It is an advantage of embodiments of the present invention that a good positioning of a template grid can be achieved using an optimization procedure that is separable from a treatment planning based on the determined position, thus reducing the overall complexity of the planning and keeping the global treatment planning tractable. It is an advantage that using a template grid limits the possible positions of the interventional tool(s) to be considered in planning, thus reducing complexity, while providing a good set of available positions to use in planning. It is an advantage that direct searches or similar optimization strategies can be used in the planning due to the finite number of available positions, which can allow a global optimum of the treatment to be found, e.g. avoiding suboptimal (locally optimal) solutions.

It is an advantage of embodiments of the present invention that reliance on the experience of a clinician is reduced. For example, an automated positioning approach as disclosed herein may increase the efficiency of clinical personnel, may avoid problems due to limited experience of a physician in training, may increase efficacy of treatments, and/or may increase reproducibility, e.g. avoiding unnecessary variability due to manual positioning of the template grid. By reducing variability across treatments due to positioning of the template grid, effects of other treatment variables can be more easily detected when analyzing clinical outcomes, thus aiding in procedural optimization.

Furthermore, by ensuring a good coverage of the target volume by available interventional paths, robustness of the treatment with respect to minor grid/patient movements can be increased. For example, a dose (radiation or ablation) can be distributed over more treatment delivery points when more suitable paths become available by a good initial positioning of the template grid, thus reducing the risk of an inaccurate delivery in only a few points.

It is an advantage of embodiments of the present invention that particularly small tumors can be effectively treated without increasing a (spatial) tolerance margin due to inaccurate positioning of the template grid. For example, a suboptimal grid positioning could tremendously hamper the quality of a delivered treatment plan, particularly for very small lesions. In a worst-case scenario, a very small lesion could be positioned fully between grid hole trajectories and thus impossible to reach by inserted catheters or needles. Such scenario would require increased margins around the target to reach the treatment volume, but at the cost of an increased dose (or thermal ablation) on nearby healthy tissues.

It is an advantage of embodiments of the present invention that a simple user interface and/or alignment procedure can be provided to position a template grid.

In a first aspect, the present invention relates to a processing device for determining a position and/or orientation for a grid template with respect to a human or animal body in a medical interventional procedure. Such grid template comprises a plurality of holes that define a corresponding plurality of grid hole trajectories, and is adapted for supporting and guiding at least one interventional tool along such grid hole trajectory when inserted through at least one of said holes into the body, in said interventional procedure.

The processing device comprises an anatomical spatial information processing unit for receiving and/or processing data representative of at least one target spatial volume in the body.

The processing device comprises a grid position sampler for generating a plurality of candidate positions and/or orientations of the grid template with respect to the at least one target spatial volume in the body.

The processing device comprises a quality calculator for calculating, for each candidate position and/or orientation of the plurality of candidate positions and/or orientations, at least one quality metric representative of the suitability of the candidate position of the grid template for the interventional procedure.

The processing device comprises a position selector for selecting a position and/or orientation from the plurality of candidate positions and/or orientations based on the at least one quality metric.

The quality calculator is adapted for determining, for each candidate position and/or orientation, a spatial relationship between each grid hole trajectory of the grid template, when positioned in accordance with the candidate position and/or orientation, with said at least one target volume. For example, the quality calculator may be adapted for determining an intersection of each grid hole trajectory of the grid template, when positioned in accordance with the candidate position and/or orientation, with said at least one target volume.

The quality calculator is also adapted for calculating the at least one quality metric, for each candidate position and/or orientation, by taking, at least, a value indicative of a geometric overlap and/or proximity of the grid hole trajectories with respect to the at least one target volume based one the determined spatial relationship and/or indicative of a treatment efficacy measure of the medical interventional procedure when constrained by the determined spatial relationship into account.

In a device in accordance with embodiments of the present invention, this value comprises a value indicative of a treatment efficacy measure of the medical interventional procedure when constrained by said determined spatial relationship, said treatment efficacy measure being representative of a radiation dose or ablation effect received in said at least one target spatial volume when one or more radiation sources or ablators are positioned along said grid hole trajectories.

For example, the treatment efficacy measure may comprise an absolute or relative (e.g. percentage of) volume of the target spatial volume that receives at least a predetermined radiation dose (e.g. at least a predetermined value in Gray units) when one or more radiation sources (e.g. emitting a predetermined radiation fluence or having a predetermined strength in units of Becquerel) are positioned along the grid hole trajectories. Similarly, the treatment efficacy measure may comprise a total, average or other summary statistic of a radiation dose received by the target spatial volume, or a predetermined volume fraction thereof, when one or more radiation sources (e.g. emitting a predetermined radiation fluence or having a predetermined strength in units of Becquere1) are positioned along the grid hole trajectories.

For example, the treatment efficacy measure may comprise an absolute or relative (e.g. percentage of) volume of the target spatial volume that receives a predetermined amount of ablation energy when one or more ablation sources (e.g. in accordance with a predetermined source power and/or dwell time) are positioned along the grid hole trajectories. For example, the treatment efficacy measure may represent an absolute or relative volume of the target spatial volume being ablated.

For example, the treatment efficacy measure may comprise an absolute or relative volume of the target spatial volume that reaches a predetermined temperature (e.g. exceeds a predetermined temperature threshold) when being ablated by one or more ablation probes when positioned along the grid hole trajectories, e.g. given a predetermined ablation power and/or dwell time and/or other predetermined ablation parameters. Likewise, the treatment efficacy measure may comprise an average, maximum, minimum or other summary statistic of the ablation energy deposited or the temperature achieved by heating by the the ablation source.

In a device in accordance with embodiments of the present invention, this value may comprise a first value indicative of a degree of intersection of (e.g. all of) said grid hole trajectories for the candidate position and/or orientation with the at least one target volume into account.

In a processing device in accordance with embodiments of the present invention, the first value may comprise a total number of intersecting grid hole trajectories with the at least one target volume, and/or a total length of the line segments that correspond to said intersections, and/or an average, or other statistical measure of central tendency, e.g. a median, of the length of said line segments that correspond to said intersections. In a processing device in accordance with embodiments of the present invention, the anatomical spatial information processing unit may be adapted for receiving and/or processing data representative of at least one spatial volume at risk in the body, e.g. a volume in the body to avoid or reduce intersection(s) with the grid hole trajectories and/or to avoid or reduce proximity of the grid hole trajectories to the volume at risk.

The quality calculator may be adapted for determining, for each candidate position and/or orientation, an intersection of each grid hole trajectory of the grid template, when positioned in accordance with the candidate position and/or orientation, with said at least one volume at risk. The quality calculator may be adapted for calculating the at least one quality metric, for each candidate position and/or orientation, by taking, at least, said first value and a second value, indicative of a degree of intersection of (e.g. all of) the grid hole trajectories for the candidate position and/or orientation with the at least one volume at risk, into account.

In a processing device in accordance with embodiments of the present invention, the second value may comprise a total number of intersecting grid hole trajectories with the at least one volume at risk, and/or a total length of the line segments that correspond to said intersections with the at least one volume at risk, and/or an average, or other statistical measure of central tendency, of the length of said line segments that correspond to said intersections with the at least one volume at risk.

In a processing device in accordance with embodiments of the present invention, the quality calculator may be adapted for calculating the at least one quality metric, for each candidate position and/or orientation, by taking, at least, said first value, optionally said second value, and a third value into account. The third value may be indicative of a minimal distance of a grid hole trajectory to the center of the, or at least one of the, at least one target volume.

In a processing device in accordance with embodiments of the present invention, the at least one quality metric may be a plurality of quality metrics, and said quality calculator may be adapted for combining said plurality of quality metrics into a composite quality metric in accordance with a weighted sum. The position selector may be adapted for selecting a position and/or orientation from the plurality of candidate positions and/or orientations for which an extremum is reached of the composite quality metric.

In a processing device in accordance with embodiments of the present invention, the at least one quality metric may be a plurality of quality metrics, and said position selector may be adapted for selecting a first subset of said plurality of candidate positions and/or orientations based on a first quality metric of said plurality of quality metrics, and for selecting at least a second subset of said first subset based on a second quality metric, different from said first quality metric, of said plurality of quality metrics, e.g. optionally further reducing the second subset via one or more nested subsets using one or more further quality metrics.

A processing device in accordance with embodiments of the present invention may comprise a user interface for receiving a prioritization configuration from a user to select an ordered set or subset of said plurality of quality metrics, in which the position selector may be adapted for selecting the first quality metric and the second quality metric in accordance with said ordered set or subset.

In a processing device in accordance with embodiments of the present invention, the grid position sampler may be adapted for generating the plurality of candidate positions and/or orientations by translating and/or rotating a position and/or orientation representation of the grid template over a plurality of different translation and/or rotation steps.

In a processing device in accordance with embodiments of the present invention, the grid position sampler may be adapted for said translating and/or rotating over the plurality of different translation and/or rotation steps relative to an initial position and/or orientation.

A processing device in accordance with embodiments of the present invention may comprise a user interface for receiving said initial position and/or orientation from a user.

In a processing device in accordance with embodiments of the present invention, the grid position sampler may be adapted for determining said initial position and/or orientation by:
- projecting the at least one target spatial volume in the body onto a plane exterior to the body and/or tangent to the surface of the body, and determining the initial position as a center of said projection; or
- determining said initial position by projecting a center of the at least one target spatial volume in the body onto a plane exterior to the body and/or tangent to the surface of the body.

In a processing device in accordance with embodiments of the present invention, the anatomical spatial information processing unit may comprise an input port for receiving said data in the form of at least one segmented medical image in which the at least one target spatial volume in the body and/or said at least one spatial volume at risk in the body are represented by corresponding segmentation labels.

In a processing device in accordance with embodiments of the present invention, the anatomical spatial information processing unit may comprise an input port for receiving said data in the form of at least one surface mesh and/or parametric spatial descriptor of the at least one target spatial volume in the body and/or the at least one spatial volume at risk in the body.

In a processing device in accordance with embodiments of the present invention, the anatomical spatial information processing unit may comprise an input port for receiving said data representative of said at least one target spatial volume in the body and/or said at least one spatial volume at risk in the body, said data being in the form of at least one medical image, and an image segmentation unit (3) for segmenting said at least one medical image such as to determine the at least one target spatial volume in the body and/or the at least one spatial volume at risk in the body.

A processing device in accordance with embodiments of the present invention may comprise a grid template alignment evaluator for receiving a position signal indicative of a physical position and/or orientation of a physical grid template, and for providing a feedback signal indicative of the position and/or orientation selected by the position selector and/or of the physical position and/or orientation and/or a relative position and/or orientation between said physical position and/or orientation and said selected position and/or orientation.

In a processing device in accordance with embodiments of the present invention, the grid template alignment evaluator may be adapted for concomitantly visualizing, using a user interface, the physical position and/or orientation and the selected position and/or orientation. In a processing device in accordance with embodiments of the present invention, the user interface may be adapted for displaying said physical position and/or orientation and said selected position and/or orientation with different display styles.

In a processing device in accordance with embodiments of the present invention, the user interface may be adapted for displaying a substantial alignment of said physical position and/or orientation and said selected position and/or orientation with a further and different (from the aforementioned styles) display style.

In a processing device in accordance with embodiments of the present invention, the feedback signal may comprise an audio signal to indicate a measure of discrepancy between the selected position and/or orientation and the physical position and/or orientation.

In a processing device in accordance with embodiments of the present invention, the feedback signal may comprise an actuator signal for controlling one or more actuators adapted for positioning said physical grid template.

In a second aspect, the present invention relates to a clinical workstation comprising a device in accordance with embodiments of the first aspect of the present invention.

In a third aspect, the present invention relates to a computer-implemented method for determining a position and/or orientation for a grid template with respect to a human or animal body in a medical interventional procedure, wherein said grid template comprises a plurality of holes that define a corresponding plurality of grid hole trajectories and wherein the grid template is adapted for supporting and guiding at least one interventional tool along such grid hole trajectory when inserted through at least one of said holes into the body in said interventional procedure. The method comprises receiving and/or processing data representative of at least one target spatial volume in the body, generating a plurality of candidate positions and/or orientations of the grid template with respect to the at least one target spatial volume in the body, and determining, for each candidate position and/or orientation, a spatial relationship between each grid hole trajectory of the grid template, when positioned in accordance with the candidate position and/or orientation, and said at least one target volume, e.g. an intersection of each grid hole trajectory with the at least one target spatial volume. The method further comprises calculating, for each candidate position and/or orientation of the plurality of candidate positions and/or orientations, at least one quality metric representative of the suitability of the candidate position of the grid template for the interventional procedure by taking, at least, a value indicative of a treatment efficacy measure of the medical interventional procedure when constrained by said determined spatial relationship into account, said treatment efficacy measure being representative of a radiation dose or ablation effect received in said at least one target spatial volume when one or more radiation sources or ablators are positioned along said grid hole trajectories, and selecting a position and/or orientation from the plurality of candidate positions and/or orientations based on said at least one quality metric.

This calculating of the at least one quality metric may take a value indicative of a geometric overlap and/or proximity of the grid hole trajectories with respect to the at least one target volume based on the determined spatial relationship into account.

For example, the at least one quality metric may be calculated by taking, at least, a first value indicative of a degree of intersection of said grid hole trajectories for the candidate position and/or orientation with the at least one target volume into account, and selecting a position and/or orientation from the plurality of candidate positions and/or orientations based on said at least one quality metric.

In a fourth aspect, the present invention relates to a computer program product, comprising executable computer program code, for implementing the computer-implemented method in accordance with embodiments of the present invention, when executing the computer program product (the executable computer program code) on a processor (e.g. on a computer device).

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a device and system in accordance with embodiments of the present invention.
Fig. 2 shows intersection segments of grid hole trajectories, e.g. treatment delivery paths, with a target volume of interest, e.g. a cancer lesion, for illustrating embodiments of the present invention.
Fig. 3 shows two different candidate positions of a grid template, and smallest distances between grid hole trajectories and the geometrical centers of volumes of interest, for illustrating embodiments of the present invention.
Fig. 4 shows visualizations of a physical grid position and a selected grid position (i.e. target position), for illustrating embodiments of the present invention.
Fig. 5 shows a grid template mounted on a stepper device, for illustrating embodiments of the present invention.
Fig. 6 shows a table mount for supporting a grid template and stepper device, for illustrating embodiments of the present invention.
Fig. 7 shows controllable positions and orientations of a grid template using a (e.g. manually controllable) stepper device, for illustrating embodiments of the present invention.
Fig. 8 illustrates a method in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a processing device for determining a position of a grid template in a medical interventional procedure, in which the grid template is used for supporting and guiding at least one interventional tool, such as a needle, an ablation tool, a brachytherapy source or a catheter, through at least one selected hole of the grid template into an anatomical volume of interest.

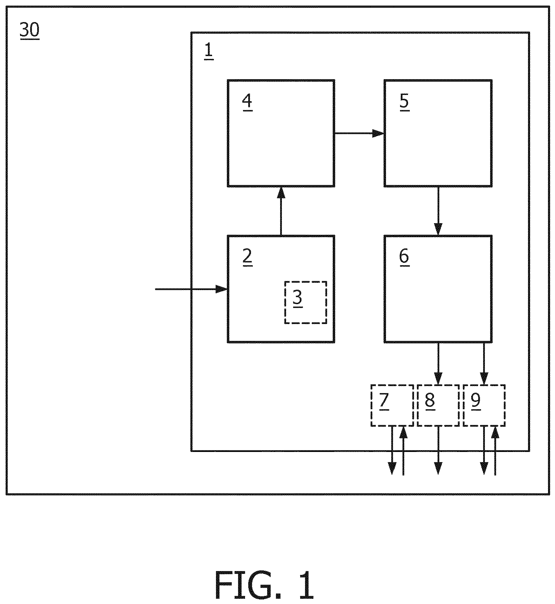

Fig. 1 illustrates an illustrative device 1 in accordance with embodiments of the present invention. The device may comprise a computer, e.g. specifically programmed for implementing the device as described. Such computer may comprise inputs and outputs, e.g. communication interface(s) for receiving data and sending data, e.g. via a data carrier and/or communication network interface. Such inputs and outputs may also comprise user interface hardware, e.g. a human interaction device for receiving input from a human user (e.g. a keyboard, a mouse, a voice interpreter, a touch interface, a gyroscope or accelerometer, etc.), a monitor for presenting information to the user, a speaker, a printer for rendering information onto a physical carrier, such as paper, a three-dimensional printer for generating a three-dimensional physical model of data, and other such elements as known in the art.

The computer may comprise a general purpose processor for carrying out instructions, e.g. a computer code, and a memory for storing such instructions. The computer may comprise a memory for storing data, e.g. for manipulating in accordance with the instructions. The device is not necessary limited to a general-purpose computer, but may also comprise an application specific integrated circuit (ASIC) and/or configurable processing hardware, e.g. a field-programmable gate array (FPGA). Furthermore, the device may be comprised in a single processing device, e.g. a computer, but also distributed over a plurality of such devices that are operably connected to each other, e.g. such that the processing described herein is carried out by the joint action of a server and a client device, or of a parallel processing system, such as a computing cluster.

The processing device 1 is adapted for determining a position of a grid template in a medical interventional procedure. Such medical interventional procedure may be a focal treatment, a biopsy, or another medical intervention to be performed by inserting the interventional tool specifically into the spatial volume. For example, the medical interventional procedure may comprise a biopsy or pathologic mapping of an anatomical region, e.g. a lesion or (part of an) organ or tissue that is suspected of being inflicted by a disease or disorder. For example, a focal treatment may refer to a percutaneous focal treatment, e.g. a percutaneous focal cancer treatment, such as a brachytherapy cancer treatment, an ablation procedure, e.g. cryoablation, thermal ablation, microwave ablation, (focused) ultrasound ablation, radiofrequency ablation, laser ablation and the like, or a treatment in which a focal source of energy is used to activate sensitized tissue and/or cells, e.g. photodynamic therapy. For example, a brachytherapy or ablation modality may be chosen by a physician in order to treat a tumor, e.g. depending on the available means, lesion size and location, affected organ, etc.. However, it is common in such procedures to place a grid template manually on (or at least near) the surface of the skin, and to insert the interventional tool (e.g. a biopsy needle, an ablator, a needle or catheter preloaded or loadable with brachytherapy seeds, ...) through one or more guiding holes of the grid template, typically selected from a larger number of possible (selectable) holes, into the body. Another example of such procedure may be an implantation of a microdevice, e.g. microstimulator, or other structure, e.g. a timed release capsule for releasing a medical compound, into a specific spot in the body.

The grid template may also be referred to as a leading grid frame or treatment grid. The grid template may be rigid or flexible grid template, as known in the art. The grid template may be radio-opaque, e.g. to avoid interference with ionizing radiation used in imaging of the patient when the grid template is positioned for the procedure. In the present disclosure, terms such as 'grid', 'template', 'template grid' and 'grid template' are considered equivalent and interchangeable, i.e. refer to the same such leading grid frame for use in an interventional procedure. The grid template comprises a plurality of holes, i.e. through holes, at different positions on the grid template. For example, the grid template may have a major surface over which such holes are distributed, such that each hole protrudes through the grid template from the major surface to a surface opposite of the major surface. For example, the grid template may have one dimension component that is substantially smaller than the other two (Cartesian) dimension components, such that typically two "major" surfaces, at opposite sides of the template, may extend over the two larger dimensions. One such major surface may be adapted for contacting (or being oriented toward) the skin of the patient, in use, while the opposite major surface allows access to these holes, such that tools, e.g. needles, pins, catheters and other such elongate elements, can be inserted from this side to the contacting side and, further along this path, into the body of the patient in a specific position (the position of the hole) and direction (e.g. as obtained by guiding the tool by the hole). Without limitation thereto, the grid template may comprise such holes arranged in rows and columns (hence "grid", e.g. a rectangular grid). This does not exclude other distribution patterns of the holes, e.g. a polar grid, a hexagonal grid. The distribution pattern may be regular or irregular, and may be uniformly or inhomogeneously distributed. Such template may be disposable or reusable. The template may be rigid, flexible, or may have a flexibility that is somewhat limited. The grid holes may be spaced at, for example, 1 mm to 20 mm distances with respect to each other (i.e. to neighboring holes). A typical example is a template with an inter-hole distance in the range of 2 mm to 10 mm, e.g. 3 mm to 8 mm, e.g. 5 mm. The holes may have a diameter to fit a predetermined needle gauge, e.g. a needle gauge in the range of 14 gauge to 18 gauge (without limitation thereto), e.g. 14g, 17g or 18g. The number of holes may range (without limitation) from e.g. 9 to 1000, e.g. typically in the range of 25 to 225, e.g. 25, 36, 49, 64, 81, 100, 121, 144, 169 or 225 holes. While these examples are based on a regular grid of equal number of rows and columns, it shall be understood that, in embodiments, the grid is neither required to be a regular rectangular grid nor to have a number of rows that is equal to the number of columns.

The grid template comprises a plurality of holes (through-holes) that define a corresponding plurality of grid hole trajectories for needles, catheters or other elongate interventional tools (when inserted through the hole). The grid template may have a substantial width, such that the direction of the grid hole trajectories are constrained to a specific direction, e.g. perpendicular to the surface of the grid template (which may or may not take a local surface curvature into account, e.g. in case of a flexible grid template). However, embodiments are not necessarily limited thereto. For example, other means may be used to control the direction of insertion, or the holes may be provided at an angle that differs from a right angle with respect to the surface.

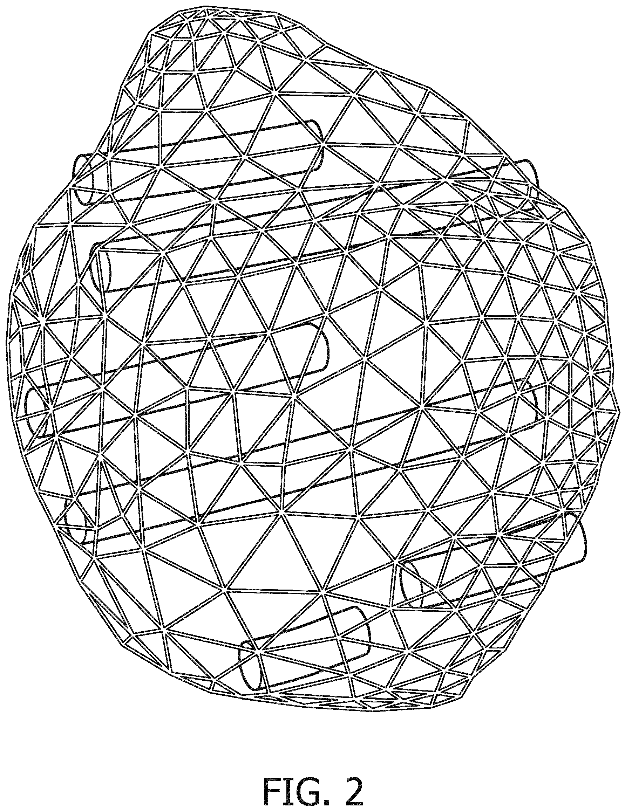

The skilled person will understand that the positioning of the grid template can be important for the success of the procedure. Typically, and if not differently indicated by a medical expert, only grid hole trajectories that intersect with (e.g. enter to) a target volume, e.g. a lesion to be treated, can be used for the interventional procedure, e.g. for treatment delivery. For example, in brachytherapy inverse planning methods, the target lesion intersection segments, e.g. as shown in Fig. 2, may be used to sample the set of dwell positions at which the radioactive source could dwell for a given optimal time to be determined. Therefore, both automatic inverse and manual forward planning solutions may benefit from an optimal leading grid position. For example, the needle tracks through the grid holes preferably intersect as much as possible with the segmented anatomy. The success and efficacy of a treatment, and the prevention of post-treatment complications, may be particularly sensitive to the initial grid position for very small lesions to be treated.

The device 1, in use thereof, determines a position of the grid template to use in such procedure, e.g. an optimal (or at least good) placement of the grid template to allow the spatial volume to be reached by inserting the interventional tool(s) through one or more holes in the grid template. For example, this position may depend on the size and location of the volume, e.g. a tumor position and size, parameters of the grid template, e.g. specifications thereof as can be provided by the grid template manufacturer. By automatically optimizing the grid template position, reliance on the experience of a physician can advantageously be reduced, and efficiency of the physician can be increased by reducing or avoiding the time spent on manually determining a suitable position. For example, in a conventional approach, the physician may be required to position the grid template, choose a set of tool positions and determine a corresponding set of tool parameters. Once the grid template is positioned, solutions are known in the prior art to automatically (or in a computationally assisted manner) select the tool positions on grid hole trajectories provided by the grid template and determine suitable parameters. For example, a manual forward planning or an automatic inverse planning solution may be applied to find the optimal set of delivery parameters (e.g. brachytherapy catheters positions, thermal ablation probe positions, dwell times, ablation time/powers values, etc.).

The processing device 1 is adapted for, e.g. programmed for, e.g. configured for, receiving spatial data representative of at least one target spatial volume in the body of a patient, into which the at least interventional tool is to be inserted via at least one hole of the grid template in accordance with the medical interventional procedure.

The processing device may also be adapted for, e.g. programmed for, e.g. configured for, receiving spatial data representative of at least one spatial volume at risk in the body of the patient that the at least interventional tool should avoid, e.g. stay clear from by a distance margin and/or should not pierce along its insertion path. The at least one target spatial volume and optionally the at least one spatial volume at risk may be, for convenience, collectively referred to as 'volume(s) of interest' further hereinbelow.

Thus, the processing device 1 comprises an anatomical spatial information processing unit 2 for receiving and/or processing data representative of at least one target spatial volume in the body of the patient; and optionally also at least one spatial volume at risk in the body. The spatial information processing unit may be or comprise an input port for receiving said data, e.g. via a digital communication network, a physical data carrier or a purpose-specific interface with a medical imaging device, e.g. a bus interface. The data may be received in the form of a medical image, e.g. a volumetric three-dimensional image of at least a part of the body that comprises the target spatial volume and/or the spatial volume at risk. Such medical image may comprise a (e.g. three-dimensional or tomographic) image obtained by X-ray computed tomography (CT), magnetic resonance imaging, nuclear medicine imaging, echography, or other medical imaging modalities (e.g. elastography, optical CT, photoacoustic imaging, magnetic particle imaging, without being limited to these examples). The medical image may be processed to define the volume(s) of interest, e.g. in an image overlay annotating voxels assigned to the volume(s) of interest, or the medical image may consist of a definition of the volume(s) of interest, i.e. may be a segmented image. Alternatively, the data may be received in the form of a descriptor of the volume(s) of interest, such as a surface mesh(es) defining the volume(s) of interest, e.g. a set of vertices, edges and faces that define the or each volume of interest as a polyhedral object in a reference coordinate space. Such faces may comprise triangles, quadrilaterals and/or other polygons (n-gons; e.g. convex polygons, or even concave polygons). The descriptor may also be defined in another suitable format, such as a parametric definition of the (each) volume(s) of interest, e.g. as parameters of one or more spheres, cubes, non-uniform rational basis splines (NURBS), or other parametric shapes, or such as a volumetric mesh, e.g. comprising also explicit volume information. While the descriptor of the volume(s) of interest may typically define the volume (or its enclosing surface) directly, a stochastic or fuzzy definition of the volume is not necessarily excluded (e.g. a probability map).

The anatomical spatial information processing unit 2 may also comprise a (volumetric) image segmentation unit 3 to segment the medical image such as to delineate the volume(s) of interest in the medical image, e.g. where the data does not yet comprise segmentation information or another form of definition of the volume(s) as referred to hereinabove. Suitable segmentation algorithms are known in the art, and may include manual segmentation (e.g. using a suitable user interface 7), semi-automatic segmentation and/or automatic segmentation. Without limitation, some examples of such segmentation approaches known in the art include voxel value thresholding, clustering methods, histogram-based methods, edge detection, region-growing methods, partial differential equation-based methods, variational methods, graph partitioning methods, watershed transformation-based methods, multi-scale methods, machine learning-based methods (e.g. trainable methods), and/or any combination thereof.

Thus, the processing device may receive a definition of the volume(s) of interest, or may otherwise determine such definition by processing of the received data, e.g. image data, which may already contain such information in an implicit sense. Therefore, all regions of interest (such as target lesions and risk organs) may be segmented in a preprocessing step, e.g. supplied to the device, by processing performed by the device (e.g. based on received image data) or by a combination of both (e.g. an initial segmentation may be supplied and refined by automatic, interactive or manual procedures performed by the device).

The processing device comprises a grid position sampler 4 for generating a plurality of candidate positions of the grid template with respect to the volume(s) of interest, e.g. in a (implicitly or explicitly) shared reference coordinate system. Generating the plurality of candidate positions may comprise translating and/or rotating the grid template over a plurality of different translation and/or rotation steps. For example, the grid position sampler may sample the position (and optionally orientation) of the grid template over set of translation steps, and/or rotation angles. An initial position may be automatically determined and/or specified by a user, e.g. using a user interface 7 of the device. For example, the user may provide a best guess for the initial grid position (x₀,y₀) based on the patient anatomy and/or prior knowledge and/or experience to initialize the positioning method performed by the device. A user-defined initial position may be verified by the device, e.g. to check whether it allows to fully encompass all target volumes, e.g. defined cancer lesions. While the term 'sampling' is used, this sampling may be a deterministic determination of candidate positions, e.g. by direct enumeration, or a stochastic sampling, e.g. by random sampling from a distribution function for the candidate position. References to 'position' may relate to position coordinate(s), to angular orientation coordinate(s) and/or combinations thereof. Thus, 'position' should not be construed narrowly as only a point in space, but may also relate to one or more angles or orientation or a combination of a point in space and an orientation, e.g. a localized vector (e.g. "localized" as opposed to a free or unbound vector).

The initial position may be automatically determined by using a fixed predetermined position, or by a simple heuristic, such as a projection of the target volume of interest onto the surface of the skin of the body, e.g. along a fixed coordinate axis or along a surface normal of the skin surface. For example, the initial grid position may be determined automatically by the geometrical barycenter of the target volume, e.g. of a single cancer lesion, projected by a two-dimensional axial projection onto the grid x,y plane (which may e.g. be defined by a plane parallel to the surface of the skin, or an approximation thereof). Likewise, in case of a plurality of target volumes, e.g. multiple lesions, a spatial average position of such projection barycenters, calculated for each target volume, may be used. Alternatives can be easily envisioned, such as using other measures of centrality, or using the barycenter of the convex hull of the plurality of target volumes. Such automatic determination of the initial position may also be executed when an initial position provided by a user is considered unsuitable, e.g. if it allows insufficient access to the target volume(s), cf. the verification mentioned hereinabove.

Translation steps may be determined by direct and deterministic enumeration, e.g. using integer (e.g. signed) multiples of a predetermined step size, in one, two or three coordinates. Such step size (or step sizes for different translation and/or rotation components) may be predetermined, or may be configurable by a user, using a user interface 7.

For example, a horizontal step size sₓ and a vertical step size s_{y} (which could be equal) may be used (predetermined or specified by a user using a user interface 7) to define the candidate positions relative to the initial position x₀,y₀ as [x,y]=[x₀+i.sₓ,y₀+j.s_{y}] for all combinations of i = { -K,-K+1...,0,..., K-1,K} and j = { -L,-L+1,-L+2,...,0,...,L-2,L-1,L}, for covering a predetermined area range 2K.sₓ by 2L.s_{y}. Consideration of additional steps in the z direction perpendicular to the x,y plane (or more accurately, steps comprising a z-component that is not constant over all considered steps) are not necessarily excluded (nor necessarily included). Likewise, rotations can be considered, e.g. using an angular step size s_{θ} for rotations in the x,y plane. Rotations may also be considered out of the x,y plane, e.g. using two or even three complementary angular components. Thus, a discrete (finite) set of positions (and/or orientations) of the grid template can be generated that span 1, 2, 3, 4, 5 or even 6 (roto)translational components of transformation. To obtain a good position estimate, at least two components, e.g. two complementary components of translation, may be preferable (even though even optimization in a single direction could be useful for some embodiments), e.g. the x and y component. For finding a position that may allow even further optimization, a third component may be taken into account as well, such as a rotation in the x,y plane. Likewise, including additional degrees of freedom may allow to obtain even better optimized position estimates. However, embodiments may be designed taking a trade-off between optimization quality and complexity into account. For example, the complexity associated with the number of sampled positions may scale as a power O(n^{k}), with k the number of degrees of freedom considered in the transformation steps.

While this processing step of generating the plurality of candidate positions is described as a separate step, in will be understood by the skilled person that iterating over the plurality of candidate positions can also be performed inline, e.g. the positions/orientations need not be calculated beforehand and stored for use in a further processing step, but may also be determined "on-the-fly" in that further processing step.

While an example was given of a uniform grid sampling of the coordinates (possibly including a rotation component), it will be understood that embodiments are not necessarily restricted to uniform sampling. For example, a polar sampling may be used to consider more positions near the initial position than further away. Alternatively, a stochastic sampling of the position space may be used, or variable step sizes, e.g. to increase sampling density near the initial position. This position (/orientation) sampling process may comprise a sampling over a horizontal ("x") and a vertical ("y") position, which may, for example, define a plane perpendicular to the grid template in its initial position, e.g. chosen or determined to be tangential to the surface of the skin of the patient at (or near) the initial position (without necessarily being limited to tangential orientations).

Displacements are also not necessarily limited to rectilinear displacements (and/or their corresponding rotations), but may also take a curvature of the surface of the body into account. For example, sampled positions (e.g. uniform Cartesian sampled positions) may be mapped onto the surface of the body to constrain the sampled positions to positions where the grid template is placed in contact with the skin. For example, when a flexible grid template is used, the positions may be sampled across a predetermined region of the skin, possibly including a rotational component in the (curved) plane. However, even for a rigid template, the orientation of the grid template may follow the contour of the body, such that positions can still be sampled over the surface of the skin, with the caveat that it may be taken into account that the rigid template might not contact the skin at all points of the grid template (nor can intersect with the skin surface) due to its rigidity and the curvature of the skin. The grid position sampler may be adapted to take this into account, for example by sampling positions (and/or relative orientations) over the surface of the skin (which may be determined in a segmentation step based on the imaging data) and adjusting each position, e.g. in a direction perpendicular to the surface of the skin, by the minimal distance required to avoid collision (i.e. intersection of a model of the rigid template with the body). Alternatively or additionally, an adjustment of the orientation may also be determined at this (each) position to avoid collision. For a flexible grid template, it may be assumed that the grid template is sufficiently flexible to conform to the surface of the skin, or a degree of flexibility may be taken into account to reject positions in which the curvature of the skin would be too strong or to determine a normal displacement (and/or orientation adjustment) that avoids collision when the grid template is maximally flexed to conform as best as possible to the local curvature within its physical tolerance margins for flexing.

The device 1 comprises a quality calculator 5 for calculating, for each candidate position of the plurality of candidate positions, at least one quality metric representative of the suitability of the candidate position of the grid template for the interventional procedure. Reference to "quality" calculator is only made for ease of disclosure, e.g. to avoid confusion with other features, and is not intended to imply anything more than what is described as the performed function of this component. Even though reference is made to a quality metric, which for example may generally increase in value if the suitability of the position is higher, a cost function, e.g. which may generally decrease if the suitability increases, is considered entirely equivalent, and therefore also as a "quality metric". The quality metric may also be referred to as an objective or objective function.

The quality calculator 5 is adapted for determining, for each candidate position and/or orientation, a spatial relationship between each grid hole trajectory of the grid template, when positioned in accordance with the candidate position and/or orientation, with the at least one (e.g. with each) target volume.

For example, the quality calculator 5 may be adapted for determining an intersection of each grid hole trajectory with the or each target volume, e.g. as illustrated in FIG. 2. The quality calculator may also determine a spatial relationship between each grid hole trajectory and the or each volume of risk, e.g. an intersection of each grid hole trajectory with the or each volume at risk.

For example, the quality calculator may comprise a model of the grid template that is parameterized to be evaluated as function of the position (e.g. each candidate position) and/or orientation of the grid template as parameter, in the coordinate space in which the volume(s) of interest is specified (or applying trivial coordinate transformations to allow the determination of said intersections). While reference is made to a "model", it shall be appreciated that such model could be particularly simple, e.g. a set of localized vectors defining each grid hole trajectory with respect to a local coordinate frame, where transformation of this local coordinate frame in accordance with the position/orientation under evaluation allows to determine the grid hole trajectories for each position/orientation, and hence also to determine said intersections. It shall also be appreciated that such model may also be considerably more complex, e.g. taking deformation properties of the grid template into account. It shall be understood that the quality calculator may comprise a plurality of models of different grid templates, or a model of a grid template that is specified in a form that is dependent on grid template parameters, such as grid hole spacing, grid configuration, dimensions of the grid etc.. A user interface 7 may be used to select the model corresponding to a grid template intended for use in the procedure, and/or to configure such grid template parameters (even though embodiments wherein only a single fixed grid template model is implemented are not necessarily excluded).

Each grid hole trajectory corresponds to a line segment of a line through a hole of the grid template in a predetermined direction with respect to the orientation of the grid template, e.g. the direction in which the hole pierces the template, e.g. perpendicular to a major surface of the grid template. Thus, the predetermined direction may correspond to the direction that an interventional tool, e.g. a needle or catheter (or comprising a needle or catheter; without limitation to other types of generally elongate interventional tools for inserting into the body), is guided in by the grid template when inserted through the hole. Therefore, the intersection may correspond to a line segment of such line where it coincides with the volume of interest. The calculated intersection may comprise an explicit identification of such line segment, but may also, additionally or alternatively, comprise a value derived therefrom, such as a length of the line segment, or merely an Boolean indicator to indicate whether the grid hole trajectory intersects with the volume of interest, e.g. indicating if an intersection exists, e.g. indicating whether the length of the line segment is different from zero.

Calculating the at least one quality metric comprises calculating the at least one quality metric, for each candidate position and/or orientation, by taking, at least, a value indicative of a geometric overlap and/or proximity of the grid hole trajectories with respect to the at least one target volume based one the determined spatial relationship and/or indicative of a treatment efficacy measure of the medical interventional procedure when constrained by the determined spatial relationship into account.

Calculating the at least one quality metric may comprise calculating a value indicative of a treatment efficacy measure of the medical interventional procedure when constrained by the determined spatial relationship. Thus, the treatment efficacy measure may be representative of a radiation dose or ablation effect received in the (or each) at least one target spatial volume when one or more radiation sources or ablators are positioned along said grid hole trajectories.

For example, the treatment efficacy measure may comprise an absolute or relative (e.g. percentage of) volume of the target spatial volume that receives at least a predetermined radiation dose (e.g. at least a predetermined value in Gray units) when one or more radiation sources (e.g. emitting a predetermined radiation fluence or having a predetermined strength in units of Becquerel) are positioned along the grid hole trajectories. Similarly, the treatment efficacy measure may comprise a total, average, maximum, minimum, median, predetermined percentile (e.g. first and/or third quartile) or other summary statistic of a radiation dose received by the target spatial volume, or a predetermined volume fraction thereof, when one or more radiation sources (e.g. emitting a predetermined radiation fluence or having a predetermined strength in units of Becquerel) are positioned along the grid hole trajectories.

For example, the treatment efficacy measure may comprise an absolute or relative (e.g. percentage of) volume of the target spatial volume that receives a predetermined amount of ablation energy when one or more ablation sources (e.g. in accordance with a predetermined source power and/or dwell time) are positioned along the grid hole trajectories. For example, the treatment efficacy measure may represent an absolute or relative volume of the target spatial volume being ablated.

For example, the treatment efficacy measure may comprise an absolute or relative volume of the target spatial volume that reaches a predetermined temperature (e.g. exceeds a predetermined temperature threshold) when being ablated by one or more ablation probes when positioned along the grid hole trajectories, e.g. given a predetermined ablation power and/or dwell time and/or other predetermined ablation parameters. Likewise, the treatment efficacy measure may comprise an average, maximum, minimum or other summary statistic of the ablation energy deposited or the temperature achieved by heating by the the ablation source.

For example, for each candidate position/orientation, an optimal position of a radiation source or ablator, may be determined along each trajectory to determine the treatment efficacy measure, or a plurality of such positions along each trajectory may be considered, e.g. two or more, e.g. a predetermined number of, sources may be distributed along the trajectories. The device may include a treatment planner to determine a suitable treatment plan constrained by the candidate positon/orientation of the grid to determine the treatment efficacy measure. A forward or inverse planning algorithm, as known in the art, may be applied to each candidate position/orientation to determine the treatment efficacy measure. However, a simplified, e.g. approximative, treatment planning algorithm may be used to determine an approximation of the treatment efficacy measure to allow an efficient estimation of the efficacy of a treatment when using a specific position/orientation of the grid template, and, after selecting the best position/orientation based on this approximative measure, a more detailed, e.g. more accurate, treatment planning algorithm may be applied to propose a treatment plan to be implemented. Furthermore, in embodiments a simplified approach may be applied, for reasons of efficiency, that do not require a detailed treatment planning to gauge the suitability of the candidate positions/orientations, e.g. based on an measure of intersection of the trajectories with the target volume(s). Such approach allows, advantageously, to determine a suitable configuration of the grid template without requiring detailed knowledge of the procedure to be applied, e.g. may be applied regardless of the specific procedure to be performed by relying solely on geometrical considerations. Nonetheless, as already mentioned, more complicated approaches that advantageously use knowledge of the procedure to be applied are not necessarily excluded in embodiments in accordance with the present invention.

For example, calculating the at least one quality metric may comprise calculating a (at least one) target value indicative of a degree of intersection of all grid hole trajectories with the (or all) target volume(s) when the grid template is positioned (and/or oriented) in accordance with the candidate position. This target value may comprise a total number of intersecting grid hole trajectories with the target volume(s). This target value may comprise a total length of the line segments intersecting a target volume, e.g. a sum of the lengths of all such intersection line segments. This target value may comprise an average length of the line segments intersecting a target volume, e.g. the sum of the lengths divided by the number of grid hole trajectories that intersect a target volume. The at least one quality metric may be calculated such as to indicate a higher suitability of the candidate position of the grid template for the interventional procedure for corresponding higher values of this target value. Thus positions/orientations of the grid template may be preferentially selected, based on the quality metric, that maximize the total number of usable grid holes to reach a target volume, and/or that maximize the total path length over which an interventional tool can be positioned in a target volume via grid hole trajectories.

Calculating the at least one quality metric may comprise calculating a (at least one) risk value indicative of a degree of intersection of all grid hole trajectories with the (or all) volume(s) at risk when the grid template is positioned (and/or oriented) in accordance with the candidate position. This risk value may comprise a total number of intersecting grid hole trajectories with the volume(s) at risk. This risk value may comprise a total length of the line segments intersecting a volume at risk, e.g. a sum of the lengths of all such intersection line segments. This risk value may comprise an average length of the line segments intersecting a volume at risk, e.g. the sum of the lengths divided by the number of grid hole trajectories intersecting a volume at risk. The at least one quality metric may be calculated such as to indicate a lower suitability of the candidate position of the grid template for the interventional procedure for corresponding higher values of this risk value. Thus positions/orientations of the grid template may be preferentially selected, based on the quality metric, that minimize the total number of usable grid holes to reach a volume at risk, and/or that minimize the total path length over which an interventional tool can be positioned in a volume at risk via grid hole trajectories.

Calculating the at least one quality metric may comprise calculating a (at least one) centrality value indicative of a minimal distance of a grid hole trajectory to the center of the target volume(s). The distance may be an Euclidean distance, but other suitable distance metrics are not necessarily excluded, such as a Manhattan (or "taxicab") distance, Chebychev (or "chessboard" or "infinity norm") distance, or generally a Minkowski distance of any integer or non-integer (yet greater than 1) order p.

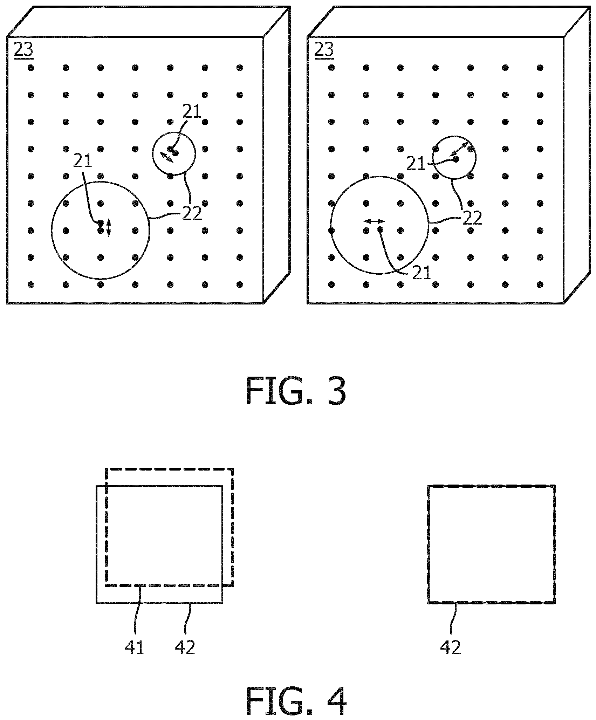

The minimum may be calculated as the minimum over all such distances calculated for all grid hole trajectories for the candidate position/orientation under consideration. These distances may be distances between the grid hole trajectory (or equivalently, the determined intersection thereof with a target volume) and the center of the target volume, in which this center may be a geometrical center, a barycenter, a centroid, a centerpoint, a center of mass, a Chebyshev center, a center of a convex hull of the target volume, a center of a minimal enclosing ball around the target volume, or another geometrical measure of centrality of a spatial volume. When multiple target volumes are defined, the center may refer to the geometrical center (or other measure of centrality) of the collective (union of) target volumes, or this minimal distance may be calculated for each target volume separately. For example, the centrality value may be indicative of the mean, median, average, or similar measure, of the minimal distances obtained for the target volumes. Alternatively, the centrality value may be indicative of the maximum, over all target volumes, of the minimum distance between a grid hole trajectory and the measure of centrality of the target volume. Thus, positions/orientations of the grid template may be preferentially selected, based on the quality metric, that minimize the closest distance to the center of the (or each) target volume that can be reached via a grid hole trajectory. Therefore, a selection of a grid position/orientation is encouraged that maximizes the probability to have grid holes trajectories passing through the center of the target region(s). This may improve symmetry of an optimized interventional procedure plan with respect to the shape of the lesion(s) at hand, and may improve robustness against minor patient/couch shifts during execution of the interventional procedure. For example, FIG. 3 illustrates two different candidate positions of a grid template. The candidate position of the grid template 23, as shown on the left, can be seen to achieve a smaller average closest grid distance than the one shown on the right. In FIG. 3, the centers of gravity 21 are shown for two target volumes 22 (projected onto the plane of the grid template).

References to "target value," "risk value," and "centrality value" should be considered as merely designations to avoid confusion for the sake of clarity, and not as implying any special property other than what has been described hereinabove. Equivalently, these values could be referred to as a "first value," a "second value," and a "third value," without implying any specific order attribute by such numeric designation.

Calculating the at least one quality metric may comprise calculating a plurality of such quality metrics, such as one or more of the mentioned target values (e.g. based on number of intersection segments, total length of intersection segments and/or average of intersection segments), one or more of the mentioned risk values (e.g. based on number of intersection segments, total length of intersection segments and/or average of intersection segments) and/or one or more of the mentioned centrality values (different measures of centrality and/or approaches to taking a plurality of target volumes into account can be considered). These quality metrics may be combined into a composite quality metric, for example by computing a weighted sum of the different quality metrics. The weights of such weighted sum may be predetermined or configurable, e.g. may be received as input from a user via a user interface 7. Each quality metric may be suitably scaled, e.g. normalized, such that the weights have a comparable effect for comparable values of the weight. For example, each value forming a term of the composite quantity metric may be divided by a known or postulated maximum value thereof, such that weights can be chosen in the range of 0 to 1, indicative of respectively "irrelevant" or "not taken into account" to "maximum relevance." It is also to be noted that some of the aforementioned quality metrics should be considered as a penalty, while others as a goal, and therefore should be combined with a suitable sign, inversion of other scaling to take this into account. For example, the number of intersecting segments with a volume(s) of risk may be subtracted from the number of intersecting segments with a target volume(s), or otherwise have its effect reversed before combination in the composite quality metric (in addition to a possible normalization of these terms, as discussed hereinabove). Alternatively, or additionally, the plurality of quality metrics may be evaluated separately, for example, first selecting suitable positions/orientations based on an extremum reached for a first quality metric, and then using a second (third, ...) quality metric to further reduce the number of selected positions/orientations to eventually arrive at only one (or a few) suitable positions/orientations. Particularly, some of the illustrative quality metrics discussed hereinabove may render integer values, e.g. a number of intersections, such that a plurality of candidate positions/orientations may render the same value for such discrete quality metric, e.g. the maximum (or minimum) of the value. Likewise, for non-discrete (continuous) quality metrics, a (predetermined or configurable) tolerance threshold may be considered to select a plurality of positions/orientations that are deemed sufficiently near the extremum of the value to be considered in a further step that used another quality metric for further reducing the number of selected positions/orientations.

The device 1 comprises a position selector 6 for selecting a position and/or orientation from the plurality of candidate positions/orientations based on the at least one quality metric, e.g. for selecting a position/orientation for which an extremum (maximum or minimum) is reached for one of the at least one quality metric and/or for the composite quality metric.

For example, in embodiments where a plurality of quality metrics are separately evaluated (i.e. in case these quality metrics are not jointly evaluated as a composite quality metric), the user interface 7 may be adapted for receiving a prioritization configuration from a user to select the quality metrics to evaluate and their order of evaluation. Thus, at least a first quality metric may be selected by the user to make a first selection of the candidate positions/orientations, and at least a second quality metric may be selected by the user to make a second selection, from the candidate positions/orientations selected by the first selection. A third or even further quality metrics may be selected by the user to even further reduce the number of selected positions/orientations. Therefore, the selector 6 may select a position/orientation by implementing a lexicographic order method, in which a priority-ordered set of goals (quality metrics) is provided by the user. However, in other embodiments, such priority order may be fixed and predetermined, or only a single (e.g. composite) quality metric may be used.

In a first illustrative example, the selector may implement a lexicographic order method. Hereto, a priority-ordered set of goals is decided by the user, such as, for example, a top priority for achieving a maximum number of grid hole trajectories intersecting the target volume(s), and a lower priority for achieving a maximum average intersection line segment length of grid hole trajectories with the target volume(s). In such approach, the selector may first select the candidate positions/orientations that achieve the maximum number of grid hole trajectories intersecting the target volume(s). If this maximum value is achieved by a single grid hole trajectory, then this solution can be provided as output without even processing the second priority level metrics/goal. Otherwise, as a second step, within the subset of grid positions/orientations that equal achieve said maximum number of intersections, the best candidate position/orientation may be selected that also produces the maximum average intersection line segment length. As mentioned, other metrics may be selected or prioritized differently, possibly including also a third, fourth, ... metric at lower priorities. In case that, after evaluating all quality metrics in their assigned order, more than a single candidate position/orientation remains selected, one may be selected at random for outputting, or all selected configurations may be output to allow a user to decide manually which configuration to use.

In a second illustrative example, a composite quality metric may be constructed by a weighted summation of all quality metrics, e.g. the quality metrics discussed hereinabove. A user may select which quality metrics to use, and may assign corresponding importance weights. Importance weights may be relative values in the interval [0,1], with 0 indicating "no relevance", and 1 indicating "maximum relevance" (without limitation of embodiments to this specific range or even necessarily to normalized "relative" values). For example, to avoid a bias, the quality metric values used as components of the composite quality metric may be normalized, e.g. to an interval [0,1], by dividing each metric value by a known maximum value. For example, a quality metric value that is indicative of a number of grid hole trajectory intersections may be divided by the total number of grid holes (e.g. 169 for typical 13x13 high dose rate brachytherapy grid template). A quality metric value that is indicative of a total (sum) of line segment lengths may be divided by a bounding dimension of the target volume(s), e.g. a maximum length of a lesion along the interventional tool insertion direction. A quality metric that is indicative of a distance of the closest trajectory to the center of a target volume may be normalized by the grid diagonal length, or by a bounding dimension of the target volume(s), e.g. a maximum diameter or radius in a plane parallel to the grid template. Thus, the selector may select the grid position/orientation that achieves the maximum composite metric/goal value, and may output this selected configuration.

The device 1 may comprise an output 8 for outputting the position and/or orientation selected by the position selector 6, for example for further evaluation and/or use by a user via the user interface 7.

The device 1 may comprise a grid alignment evaluator 9 for receiving a position signal indicative of a position and/or orientation of the physical grid template with respect to the body of the patient, and for providing a feedback signal indicative of the position and/or orientation selected by the position selector 6.

For example, the grid alignment evaluator may visualize the position signal and the selected position on a display device, e.g. in a user interface 7, such that a user can position/orient the physical grid template such as to achieve alignment with the selected position/orientation. Thus, a real-time visual guide can be provided to help in optimal manual grid positioning. For example, a contour of the physical grid template, based on the received position signal, and a contour of the grid template corresponding to the selected position and/or orientation may both be displayed, for example using different line styles, colors, or blinking properties (e.g. one of those may be shown as a permanent shape, i.e. continuously displayed, while the other may be shown as a blinking shape, i.e. intermittently displayed). For example, the selected position may be represented by a blinking contour and/or by a dashed line contour. For example, the contour of the current (physical) grid position may be shown in a different color than the optimal (selected) grid position. In the example shown in FIG. 4, the optimal grid position is shown by a dashed line 41, while the current, manual position is shown by the solid line 42. When both contours coincide, e.g. when alignment has been achieved, this may be indicated by a suitable change in the presented image. For example, as shown on the right side in FIG. 4, the color of the solid line 42 may change from, for example, red (left illustration) to green (right illustration).

Additionally or alternatively, the feedback signal may comprise an audio signal to indicate a measure of discrepancy between the selected position and the physical position. For example, a frequency of a tone or a frequency of audio pulses may increase or decrease as function of the distance between the selected position and the physical position (and/or taking a difference in orientation into account). Thus, for example, audio pulses with increasing frequency may be used to indicate an increased vicinity to the optimal position.

Alternatively or additionally, the feedback signal may comprise an actuator signal for controlling one or more actuators adapted for positioning the physical grid template. Thus, the device may control the position of the physical grid template automatically such as to position the grid template at the selected position/orientation.

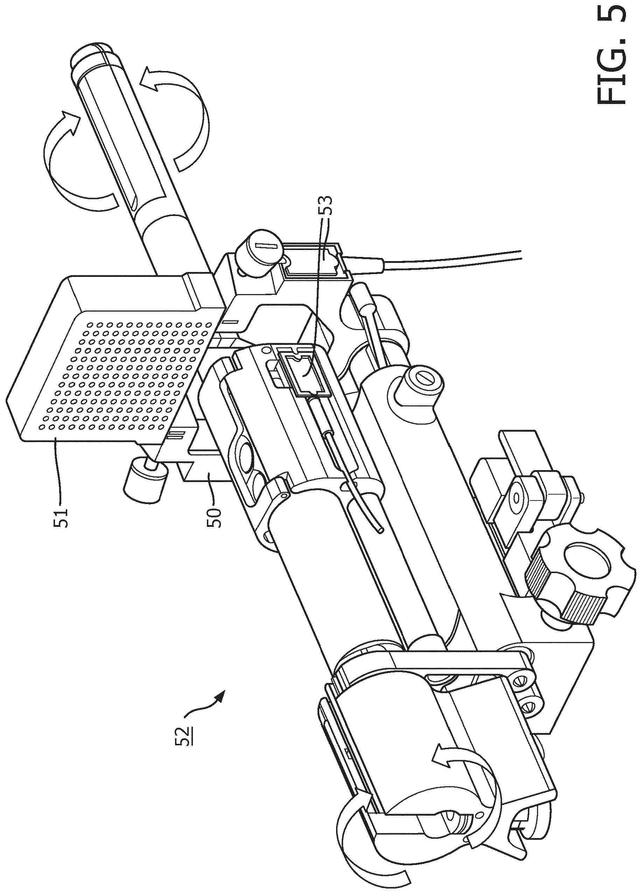

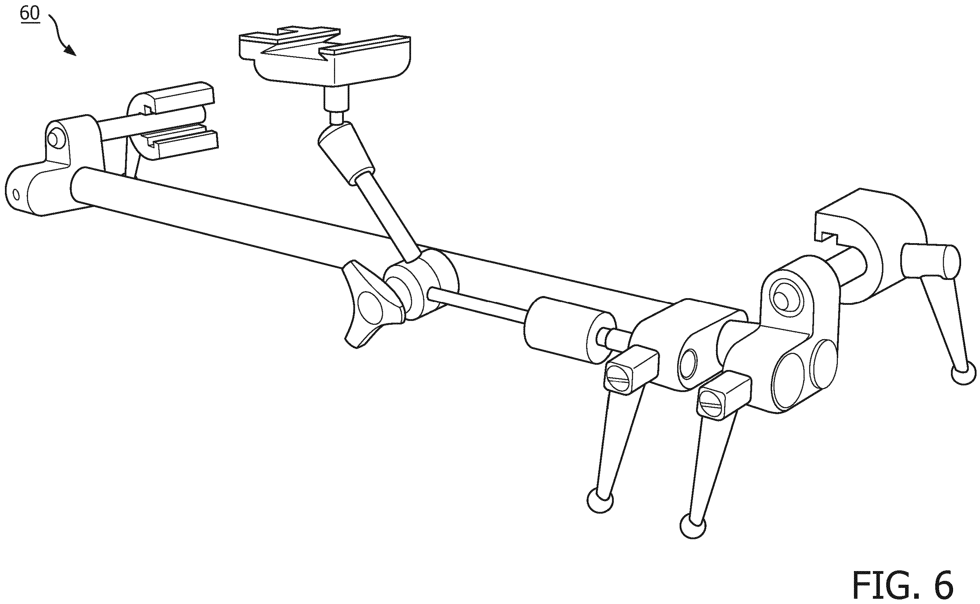

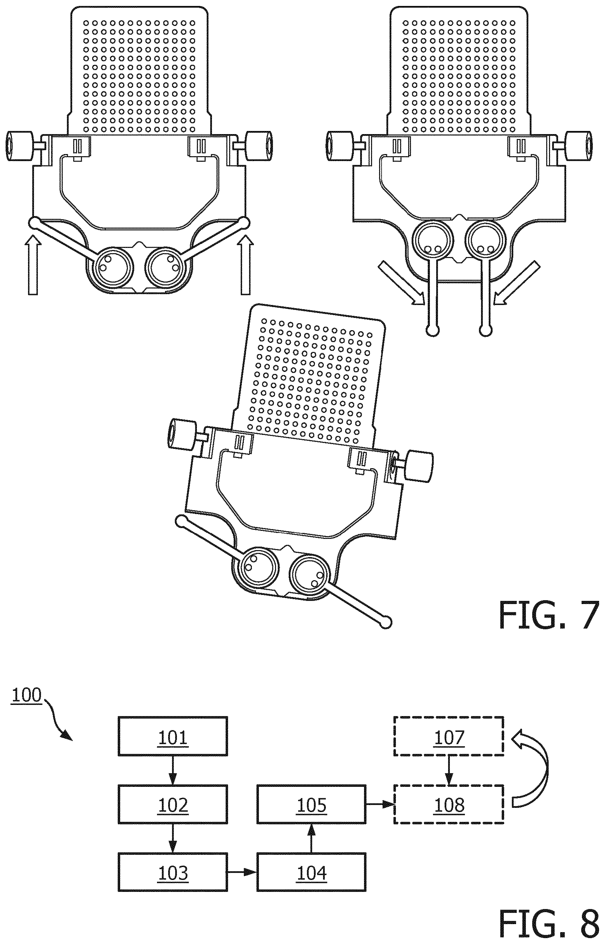

The position signal may be received from a tracking sensor 53, e.g. an electromagnetic (EM) tracking sensor integrated in, or attachable to, the grid template or to a carrier mechanism (e.g. a stepper device) for positioning the grid template. For example, the grid template 51 may be mounted on a translation and/or rotation stage 50,52,60, such as an ultrasound stepper device 50, as illustrated in FIG. 5 and FIG. 6. The ultrasound stepper device 50 may provide fine control of a translation and tilting of the grid template, as illustrated in FIG. 7. A table mount 60 (and possibly also a rotation stage 52) may allow a coarse positioning and rotation of the ultrasound stepper device 50, such that, jointly, substantial freedom in positioning and orienting the grid template can be achieved, while precision movements can be achieved in at least the degrees of freedom provided by the ultrasound stepper device.

While the position signal may be received from a tracking sensor 53, e.g. an EM tracking sensor, embodiments may also provide different means of determining the actual position and/or orientation of the physical grid template. For example, the grid template may be detected in a live imaging stream, such as fluoroscopy, optical imaging, ultrasound imaging, magnetic resonance imaging, or other modalities that allow the capturing of image information at a sufficiently high frequency, e.g. at least 5 images per minute, preferably at least 20 images per minute, even more preferably at least 1 image per second, ideally at least 20 images per second.

In a second aspect, the present invention relates to a clinical workstation 30 comprising a device 1 as described hereinabove. The clinical workstation may be adapted for presenting visual information, e.g. an imaging visualization workstation. The workstation may comprise one or more graphical display devices, e.g. monitors, a user interface device(s), such as a keyboard and/or a mouse and/or other human interface devices known in the art, and a processor. The workstation may be embodied by a computer, a smartphone, a tablet, a network server computer, and/or combinations thereof. Such clinical workstation may be suitable for or integrated in a radiology suite, a biopsy lab suite, an operating theatre suite, a radiotherapy planning and/or execution system, and the like.

Embodiments may also comprise, in addition to the device 1 and/or the clinical workstation 30, the tracking sensor 53 and/or the grid template 51 and/or the stepper device 50, e.g. as a kit of parts. Embodiments may further comprise the at least one interventional tool, e.g. a needle, a catheter, an ablation probe, a needle loaded with one or more brachytherapy seeds, etc., adapted for use with the grid template, e.g. having dimensions such as to fit, preferably in a sufficiently tight yet slidable manner, in the holes provided in the grid template.

In a third aspect, the present invention relates to a computer-implemented method for determining a position and/or orientation for a grid template with respect to a human or animal body in a medical interventional procedure, wherein said grid template comprises a plurality of holes that define a corresponding plurality of grid hole trajectories and wherein the grid template is adapted for supporting and guiding at least one interventional tool along such grid hole trajectory when inserted through at least one of said holes into the body in said interventional procedure.

Fig. 8 illustrates an exemplary computer-implemented method 100 in accordance with embodiments of the present invention.

The computer-implemented method 100 comprises receiving and/or processing 101 data representative of at least one target spatial volume in the body, and optionally also data representative of at least one spatial volume at risk in the body. For example, such data may comprise at least one segmented medical image in which the at least one target spatial volume in the body and/or said at least one spatial volume at risk in the body are represented by corresponding segmentation labels, and/or at least one surface mesh and/or parametric spatial descriptor of the at least one target spatial volume in the body and/or the at least one spatial volume at risk in the body, and/or at least one medical image. For example, processing the data may comprise segmenting the at least one medical image such as to determine the at least one target spatial volume in the body and/or the at least one spatial volume at risk in the body (even though such segmentation may alternatively be received from an external source, in accordance with embodiments of the present invention). The processing may comprise determining a surface mesh of the segmentation, or such surface mesh (or alternative descriptor) may be received form an external source.

The method 100 comprises generating 102 a plurality of candidate positions and/or orientations of the grid template with respect to the at least one target spatial volume in the body.

The method 100 comprises determining 103, for each candidate position and/or orientation, a spatial relationship between each grid hole trajectory of the grid template, when positioned in accordance with the candidate position and/or orientation, and said at least one target volume, and optionally also with said at least one volume at risk. For example, an intersection between each grid hole trajectory with the (or each) at least one target volume may be determined.

The method comprises calculating 104, for each candidate position and/or orientation of the plurality of candidate positions and/or orientations, at least one quality metric representative of the suitability of the candidate position of the grid template for the interventional procedure.

This calculating 104 comprises calculating the at least one quality metric by taking a value indicative of a geometric overlap and/or proximity of the grid hole trajectories with respect to the at least one target volume based on the determined spatial relationship into account. This calculating of the at least one quality metric may take a value indicative of a treatment efficacy measure of the medical interventional procedure when constrained by the determined spatial relationship into account.

For example, the at least one quality metric may be calculated by taking, at least, a first value indicative of a degree of intersection of said grid hole trajectories for the candidate position and/or orientation with the at least one target volume into account. The first value may comprise a total number of intersecting grid hole trajectories with the at least one target volume, and/or a total length of the line segments that correspond to said intersections, and/or an average, or other statistical measure of central tendency, of the length of said line segments that correspond to said intersections.

Calculating 104 the at least one quality metric may also take a second value, indicative of a degree of intersection of said grid hole trajectories for the candidate position and/or orientation with the at least one volume at risk, into account. The second value may comprise a total number of intersecting grid hole trajectories with the at least one volume at risk, and/or a total length of the line segments that correspond to said intersections with the at least one volume at risk, and/or an average, or other statistical measure of central tendency, of the length of said line segments that correspond to said intersections with the at least one volume at risk.

Calculating 104 the at least one quality metric may also take a third value into account, wherein said third value is indicative of a minimal distance of a grid hole trajectory to the center of the, or at least one of the, at least one target volume.

Calculating 104 the at least one quality metric may comprise calculating a plurality of metrics, such as one or more "first" values (e.g. a number, total length and/or average of said intersections), and/or one or more "second" values (e.g. a number, total length and/or average of said intersections) and/or the third value, and combining said plurality of quality metrics into a composite quality metric in accordance with a weighted sum.

The method 100 comprises selecting 105 a position and/or orientation from the plurality of candidate positions and/or orientations based on the at least one quality metric, e.g. selecting a position and/or orientation from the plurality of candidate positions and/or orientations for which an extremum is reached of one or more of the quality metrics and/or of the composite quality metric. Selecting 105 may also comprise a prioritized stepwise selection based on a plurality of said quality metrics evaluated in a predetermined or configurable order (e.g. a lexicographic order method as discussed hereinabove).

Thus, a direct search may be executed over the finite discrete set of candidate grid positions and/or orientations in order to find a (substantially) optimal position and/or orientation that maximizes the grid hole trajectory intersection with the target lesion(s), possibly taking tissue/organs at risk into account and/or possibly taking a preference for more symmetrical or at least more central distributions of the grid hole trajectories over the target lesion(s) into account.

The method may also comprise receiving 107 a position signal indicative of a physical position and/or orientation of a physical grid template.

The method may comprise providing 108 a feedback signal indicative of the selected position and/or orientation and/or of the physical position and/or orientation and/or a relative position and/or orientation between said physical position and/or orientation and said selected position and/or orientation.

For example, providing 108 the feedback signal may comprise concomitantly visualizing, using a user interface, the physical position and/or orientation and the selected position and/or orientation. For example, a current physical position of the leading grid and the determined "optimal" position can be visualized in real-time on a suitable graphic user interface (GUI) to guide a user during manual positioning of the leading grid. For example, the computed optimal grid position may be visualized in real-time on the GUI, for example as a blinking square shape (without limitation), to guide the clinical expert during manual position of the leading grid template, the position of which may be indicated in real-time, preferably using a different display style to allow both shapes to be distinguished easily.

For example, providing 108 the feedback signal may comprise generating an audio signal to indicate a measure of discrepancy between the selected position and/or orientation and the physical position and/or orientation.

For example, providing 108 the feedback signal may comprise generating an actuator signal for controlling one or more actuators adapted for positioning said physical grid template.

The method may also comprise performing a forward or inverse treatment planning of the interventional procedure using the selected position and/or orientation as input, e.g. as a predetermined parameter of the planning algorithm. Such planning algorithms are well-known in the art, and therefore not discussed in greater detail in the present disclosure. Likewise, the workstation in accordance with embodiments of the second aspect of the present invention may comprise a treatment planning system for performing said forward or inverse treatment planning.

Embodiments of the present invention may also relate to a method comprising the steps of obtaining such (physical) grid template, obtaining data representative of at least one target spatial volume in the body, using a medical imaging technique, and executing a computer-implemented method as discussed hereinabove. Thus, a position/orientation of a grid template for use in a medical interventional procedure, such as a focal treatment, a biopsy, or another medical intervention to be performed by inserting the interventional tool specifically into the target spatial volume in the body can be determined. The grid template, e.g. a leading grid frame or treatment grid, may be rigid or flexible grid template, as known in the art. The grid template may be radio-opaque (without limitation). The grid template comprises a plurality of holes, i.e. through-holes, at different positions on the grid template.

For example, such method may comprise imaging the body of a patient to provide said data.

For example, such method may also comprise determining the physical position and/or orientation of a physical grid template, e.g. using a position sensor, to provide the position signal.

Such method may also comprise actuating an actuator to position the grid template based on the feedback signal, or may comprise manually positioning the grid template by using the feedback signal, e.g. an audio/visual cue, for guidance.

Other features, or details of the features described hereinabove, of a device in accordance with embodiments of the present invention shall be clear in view of the description provided hereinabove relating to a method in accordance with embodiments of the present invention, or vice versa.

In a fourth aspect, the present invention relates to a computer program product for executing a computer-implemented method in accordance with embodiments of the present invention when executing the computer program product on a suitable processor.

## Claims

1. A processing device (1) for determining a position and/or orientation for a grid template with respect to a human or animal body in a medical interventional procedure, wherein said grid template comprises a plurality of holes that define a corresponding plurality of grid hole trajectories and wherein the grid template is adapted for supporting and guiding at least one interventional tool along such grid hole trajectory when inserted through at least one of said holes into the body in said interventional procedure, the processing device comprising:
- an anatomical spatial information processing unit (2) for receiving and/or processing data representative of at least one target spatial volume in the body;
- a grid position sampler (4) for generating a plurality of candidate positions and/or orientations of the grid template with respect to the at least one target spatial volume in the body;
- a quality calculator (5) for calculating, for each candidate position and/or orientation of the plurality of candidate positions and/or orientations, at least one quality metric representative of the suitability of the candidate position of the grid template for the interventional procedure, and
- a position selector (6) for selecting a position and/or orientation from the plurality of candidate positions and/or orientations based on the at least one quality metric,
wherein said quality calculator (5) is adapted for determining, for each candidate position and/or orientation, a spatial relationship between each grid hole trajectory of the grid template, when positioned in accordance with the candidate position and/or orientation, and said at least one target volume,
wherein said at least one quality metric comprises a value indicative of a treatment efficacy measure of the medical interventional procedure when constrained by said determined spatial relationship, **characterised in that** said treatment efficacy measure being representative of a radiation dose or ablation effect received in said at least one target spatial volume when one or more radiation sources or ablators are positioned along said grid hole trajectories.

2. The device of claim 1, wherein said value is further indicative of a geometric overlap and/or proximity of the grid hole trajectories with respect to said at least one target volume based on said determined spatial relationship.

3. The device of claim 1 or claim 2, wherein said quality calculator (5) is adapted for determining said spatial relationship by determining, for each candidate position and/or orientation, an intersection between each grid hole trajectory of the grid template, when positioned in accordance with the candidate position and/or orientation, with said at least one target volume, and for calculating the at least one quality metric, for each candidate position and/or orientation, by taking, at least, a first value indicative of a degree of intersection of said grid hole trajectories for the candidate position and/or orientation with the at least one target volume into account.

4. The device of claim 3, wherein said first value comprises a total number of intersecting grid hole trajectories with the at least one target volume, and/or a total length of the line segments that correspond to said intersections, and/or an average, or other statistical measure of central tendency, of the length of said line segments that correspond to said intersections.

5. The device of claim 3 or claim 4, wherein said anatomical spatial information processing unit (2) is furthermore adapted for receiving and/or processing data representative of at least one spatial volume at risk in the body,
wherein said quality calculator (5) is adapted for determining, for each candidate position and/or orientation, an intersection of each grid hole trajectory of the grid template, when positioned in accordance with the candidate position and/or orientation, with said at least one volume at risk,
wherein said quality calculator is adapted for calculating the at least one quality metric, for each candidate position and/or orientation, by taking, at least, said first value and a second value, indicative of a degree of intersection of said grid hole trajectories for the candidate position and/or orientation with the at least one volume at risk, into account.

6. The device of claim 5, wherein said second value comprises a total number of intersecting grid hole trajectories with the at least one volume at risk, and/or a total length of the line segments that correspond to said intersections with the at least one volume at risk, and/or an average, or other statistical measure of central tendency, of the length of said line segments that correspond to said intersections with the at least one volume at risk.

7. The device of any of claims 3 to 6, wherein said quality calculator (5) is adapted for calculating the at least one quality metric, for each candidate position and/or orientation, by taking, at least, said first value and a third value into account, wherein said third value is indicative of a minimal distance of a grid hole trajectory to the center of the, or at least one of the, at least one target volume.

8. The device of any of the claims 3 to 7, wherein said at least one quality metric is a plurality of quality metrics, wherein said quality calculator (5) is adapted for combining said plurality of quality metrics into a composite quality metric in accordance with a weighted sum, and wherein said position selector (6) is adapted for selecting a position and/or orientation from the plurality of candidate positions and/or orientations for which an extremum is reached of the composite quality metric.

9. The device of any of the previous claims, wherein said at least one quality metric is a plurality of quality metrics, wherein said position selector (6) is adapted for selecting a first subset of said plurality of candidate positions and/or orientations based on a first quality metric of said plurality of quality metrics, and for selecting at least a second subset of said first subset based on a second quality metric, different from said first quality metric, of said plurality of quality metrics.

10. The device of any of the previous claims, wherein said grid position sampler (4) is adapted for generating the plurality of candidate positions and/or orientations by translating and/or rotating a position and/or orientation representation of the grid template over a plurality of different translation and/or rotation steps.

11. The device of any of the previous claims, comprising a grid template alignment evaluator (9) for receiving a position signal indicative of a physical position and/or orientation of a physical grid template, and for providing a feedback signal indicative of the position and/or orientation selected by the position selector (6) and/or of the physical position and/or orientation of a relative position and/or orientation between said physical position and/or orientation and said selected position and/or orientation.

12. The device of claim 11, wherein said grid template alignment evaluator (9) is adapted for concomitantly visualizing, using a user interface (7), the physical position and/or orientation and the selected position and/or orientation.

13. A computer-implemented method (100) for determining a position and/or orientation for a grid template with respect to a human or animal body in a medical interventional procedure, wherein said grid template comprises a plurality of holes that define a corresponding plurality of grid hole trajectories and wherein the grid template is adapted for supporting and guiding at least one interventional tool along such grid hole trajectory when inserted through at least one of said holes into the body in said interventional procedure, the method comprising:
- receiving and/or processing (101) data representative of at least one target spatial volume in the body,
- generating (102) a plurality of candidate positions and/or orientations of the grid template with respect to the at least one target spatial volume in the body,
- determining (103), for each candidate position and/or orientation, a spatial relationship between each grid hole trajectory of the grid template, when positioned in accordance with the candidate position and/or orientation, and said at least one target volume,
- calculating (104), for each candidate position and/or orientation of the plurality of candidate positions and/or orientations, at least one quality metric representative of the suitability of the candidate position of the grid template for the interventional procedure by taking, at least, a value indicative of a treatment efficacy measure of the medical interventional procedure when constrained by said determined spatial relationship into account, said treatment efficacy measure being representative of a radiation dose or ablation effect received in said at least one target spatial volume when one or more radiation sources or ablators are positioned along said grid hole trajectories, and
- selecting (105) a position and/or orientation from the plurality of candidate positions and/or orientations based on said at least one quality metric.

14. A computer program product for implementing the computer-implemented method (100) in accordance with claim 13, when executing the computer program product on a processor.

## Patentansprüche

1. Verarbeitungsvorrichtung (1) zum Bestimmen einer Position und/oder Ausrichtung für eine Rasterschablone in Bezug auf einen menschlichen oder tierischen Körper bei einem interventionellen medizinischen Eingriff, wobei die Rasterschablone eine Vielzahl von Löchern umfasst, die eine entsprechende Vielzahl von Rasterlochbahnen definieren, und wobei die Rasterschablone zum Tragen und Führen mindestens eines interventionellen Werkzeugs entlang einer solchen Rasterlochbahn angepasst ist, wenn es bei dem interventionellen Eingriff durch mindestens eines der Löcher in den Körper eingeführt wird, wobei die Verarbeitungsvorrichtung umfasst:
- eine Verarbeitungseinheit für anatomische räumliche Informationen (2) zum Empfangen und/oder Verarbeiten von Daten, die für mindestens ein räumliches Zielvolumen in dem Körper repräsentativ sind;
- einen Rasterpositionsabtaster (4) zum Erzeugen einer Vielzahl von Kandidatenpositionen und/oder -ausrichtungen der Rasterschablone in Bezug auf das mindestens eine räumliche Zielvolumen in dem Körper;
- einen Qualitätsrechner (5) zum Berechnen, für jede Kandidatenposition und/oder -ausrichtung der Vielzahl von Kandidatenpositionen und/oder -ausrichtungen, mindestens einer Qualitätsmetrik, die für die Eignung der Kandidatenposition der Rasterschablone für den interventionellen Eingriff repräsentativ ist, und
- einen Positionswähler (6) zum Auswählen einer Position und/oder Ausrichtung aus der Vielzahl von Kandidatenpositionen und/oder -ausrichtungen basierend auf der mindestens einen Qualitätsmetrik,
wobei der Qualitätsrechner (5) zum Bestimmen für jede Kandidatenposition und/oder -ausrichtung einer räumlichen Beziehung zwischen jeder Rasterlochbahn der Rasterschablone, wenn diese in Übereinstimmung mit der Kandidatenposition und/oder -ausrichtung positioniert ist, und dem mindestens einen Zielvolumen angepasst ist,
wobei die mindestens eine Qualitätsmetrik einen Wert umfasst, der bezeichnend für einen Behandlungswirksamkeitsmesswert des interventionellen medizinischen Eingriffs ist, wenn sie durch die bestimmte räumliche Beziehung eingeschränkt ist, **dadurch gekennzeichnet, dass** der Behandlungswirksamkeitsmesswert für eine Strahlungsdosis oder einen Ablationseffekt repräsentativ ist, die bzw. der in dem mindestens einen räumlichen Zielvolumen empfangen wird, wenn eine oder mehrere Strahlungsquellen oder Ablatoren entlang der Rasterlochbahnen positioniert werden.

2. Vorrichtung nach Anspruch 1, wobei der Wert weiter bezeichnend für eine geometrische Überlappung und/oder Nähe der Rasterlochbahnen in Bezug auf das mindestens eine Zielvolumen basierend auf der bestimmten räumlichen Beziehung ist.

3. Vorrichtung nach Anspruch 1 oder Anspruch 2, wobei der Qualitätsrechner (5) zum Bestimmen der räumlichen Beziehung durch Bestimmen für jede Kandidatenposition und/oder -ausrichtung eines Schnittpunkts zwischen jeder Rasterlochbahn der Rasterschablone, wenn diese in Übereinstimmung mit der Kandidatenposition und/oder -ausrichtung positioniert ist, mit dem mindestens einen Zielvolumen und zum Berechnen der mindestens einen Qualitätsmetrik für jede Kandidatenposition und/oder -ausrichtung durch Berücksichtigen mindestens eines ersten Werts, der einen Grad der Überschneidung der Rasterlochbahnen für die Kandidatenposition und/oder -ausrichtung mit dem mindestens einen Zielvolumen angibt, angepasst ist.

4. Vorrichtung nach Anspruch 3, wobei der erste Wert eine Gesamtanzahl von sich mit dem mindestens einen Zielvolumen schneidenden Rasterlochbahnen, und/oder eine Gesamtlänge der Liniensegmente, die den Schnittpunkten entsprechen, und/oder einen Durchschnitt oder einen anderen statistischen Messwert einer zentralen Tendenz der Länge der Liniensegmente, die den Schnittpunkten entsprechen, umfasst.

5. Vorrichtung nach Anspruch 3 oder Anspruch 4, wobei die Verarbeitungseinheit für anatomische räumliche Informationen (2) weiter zum Empfangen und/oder Verarbeiten von Daten, die für mindestens ein gefährdetes räumliches Volumen in dem Körper repräsentativ sind, angepasst ist,
wobei der Qualitätsrechner (5) zum Bestimmen für jede Kandidatenposition und/oder -ausrichtung eines Schnittpunkts jeder Rasterlochbahn der Rasterschablone, wenn diese in Übereinstimmung mit der Kandidatenposition und/oder -ausrichtung positioniert ist, mit dem mindestens einen gefährdeten Volumen angepasst ist,
wobei der Qualitätsrechner zum Berechnen der mindestens einen Qualitätsmetrik für jede Kandidatenposition und/oder -ausrichtung durch Berücksichtigen mindestens des ersten Werts und eines zweiten Werts, der bezeichnend für einen Grad der Überschneidung der Rasterlochbahnen für die Kandidatenposition und/oder -ausrichtung mit dem mindestens einen gefährdeten Volumen ist, angepasst ist.

6. Vorrichtung nach Anspruch 5, wobei der zweite Wert eine Gesamtanzahl von sich mit dem mindestens einen gefährdeten Volumen schneidenden Rasterlochbahnen, und/oder eine Gesamtlänge der Liniensegmente, die den Schnittpunkten mit dem mindestens einen gefährdeten Volumen entsprechen, und/oder einen Durchschnitt oder ein anderes statistisches Maß einer zentralen Tendenz der Länge der Liniensegmente, die den Schnittpunkten mit dem mindestens einen gefährdeten Volumen entsprechen, umfasst.

7. Vorrichtung nach einem der Ansprüche 3 bis 6, wobei der Qualitätsrechner (5) zum Berechnen der mindestens einen Qualitätsmetrik für jede Kandidatenposition und/oder - ausrichtung durch Berücksichtigen mindestens des ersten Werts und eines dritten Werts angepasst ist, wobei der dritte Wert bezeichnend für einen Mindestabstand einer Rasterlochbahn zum Mittelpunkt des oder mindestens einen von dem mindestens einen Zielvolumen ist.

8. Vorrichtung nach einem der Ansprüche 3 bis 7, wobei die mindestens eine Qualitätsmetrik eine Vielzahl von Qualitätsmetriken ist, wobei der Qualitätsrechner (5) zum Kombinieren der Vielzahl von Qualitätsmetriken in Übereinstimmung mit einer gewichteten Summe zu einer zusammengesetzten Qualitätsmetrik angepasst ist, und wobei der Positionswähler (6) zum Auswählen einer Position und/oder Ausrichtung aus der Vielzahl von Kandidatenpositionen und/oder -ausrichtungen angepasst ist, bei der ein Extremwert der zusammengesetzten Qualitätsmetrik erreicht wird.

9. Vorrichtung nach einem der vorstehenden Ansprüche, wobei die mindestens eine Qualitätsmetrik eine Vielzahl von Qualitätsmetriken ist, wobei der Positionswähler (6) zum Auswählen einer ersten Teilmenge der Vielzahl von Kandidatenpositionen und/oder -ausrichtungen basierend auf einer ersten Qualitätsmetrik der Vielzahl von Qualitätsmetriken, und zum Auswählen mindestens einer zweiten Teilmenge der ersten Teilmenge basierend auf einer sich von der ersten Qualitätsmetrik unterscheidenden zweiten Qualitätsmetrik der Vielzahl von Qualitätsmetriken angepasst ist.

10. Vorrichtung nach einem der vorstehenden Ansprüche, wobei der Rasterpositionsabtaster (4) zum Erzeugen der Vielzahl von Kandidatenpositionen und/oder -ausrichtungen durch Verschieben und/oder Drehen einer Positions- und/oder Ausrichtungsdarstellung der Rasterschablone über eine Vielzahl unterschiedlicher Verschiebe- und/oder Drehschritte angepasst ist.

11. Vorrichtung nach einem der vorstehenden Ansprüche, die einen Rasterschablonenausrichtungsauswerter (9) zum Empfangen eines Positionssignals, das bezeichnend für eine physische Position und/oder Ausrichtung einer physischen Rasterschablone ist, und zum Bereitstellen eines Rückmeldesignals, das bezeichnend für die durch den Positionswähler (6) ausgewählte Position und/oder Ausrichtung und/oder die physische Position und/oder Ausrichtung einer relativen Position und/oder Ausrichtung zwischen der physischen Position und/oder Ausrichtung und der ausgewählten Position und/oder Ausrichtung ist, umfasst.

12. Vorrichtung nach Anspruch 11, wobei der Rasterschablonenausrichtungsauswerter (9) zum gleichzeitigen Visualisieren der physischen Position und/oder Ausrichtung und der ausgewählten Position und/oder Ausrichtung unter Verwendung einer Benutzeroberfläche (7) angepasst ist.

13. Computerimplementiertes Verfahren (100) zum Bestimmen einer Position und/oder Ausrichtung für eine Rasterschablone in Bezug auf einen menschlichen oder tierischen Körper bei einem interventionellen medizinischen Eingriff, wobei die Rasterschablone eine Vielzahl von Löchern umfasst, die eine entsprechende Vielzahl von Rasterlochbahnen definieren, und wobei die Rasterschablone zum Tragen und Führen mindestens eines interventionellen Werkzeugs entlang einer solchen Rasterlochbahn angepasst ist, wenn es bei dem interventionellen Eingriff durch mindestens eines der Löcher in den Körper eingeführt wird, wobei das Verfahren umfasst:
- Empfangen und/oder Verarbeiten (101) von Daten, die für mindestens ein räumliches Zielvolumen in dem Körper repräsentativ sind,
- Erzeugen (102) einer Vielzahl von Kandidatenpositionen und/oder - ausrichtungen der Rasterschablone in Bezug auf das mindestens eine räumliche Zielvolumen in dem Körper,
- Bestimmen (103), für jede Kandidatenposition und/oder -ausrichtung einer räumlichen Beziehung zwischen jeder Rasterlochbahn der Rasterschablone, wenn diese in Übereinstimmung mit der Kandidatenposition und/oder -ausrichtung positioniert ist, und dem mindestens einen Zielvolumen,
- Berechnen (104), für jede Kandidatenposition und/oder -ausrichtung der Vielzahl von Kandidatenpositionen und/oder -ausrichtungen, mindestens einer Qualitätsmetrik, die für die Eignung der Kandidatenposition der Rasterschablone für den interventionellen Eingriff repräsentativ ist, durch Berücksichtigen mindestens eines Wertes, der bezeichnend für einen Behandlungswirksamkeitsmesswert des interventionellen medizinischen Eingriffs ist, wenn sie durch die bestimmte räumliche Beziehung eingeschränkt ist, wobei der Behandlungswirksamkeitsmesswert für eine Strahlungsdosis oder einen Ablationseffekt repräsentativ ist, die bzw. der in dem mindestens einen räumlichen Zielvolumen empfangen wird, wenn eine oder mehrere Strahlungsquellen oder Ablatoren entlang der Rasterlochbahnen positioniert werden, und
- Auswählen (105) einer Position und/oder Ausrichtung aus der Vielzahl von Kandidatenpositionen und/oder -ausrichtungen basierend auf der mindestens einen Qualitätsmetrik.

14. Computerprogrammprodukt zum Implementieren des computerimplementierten Verfahrens (100) nach Anspruch 13, wenn das Computerprogrammprodukt auf einem Prozessor ausgeführt wird.

## Revendications

1. Dispositif de traitement (1) pour déterminer une position et/ou une orientation d'un modèle de grille par rapport à un corps humain ou animal au cours d'une intervention médicale, dans lequel ledit modèle de grille comprend une pluralité de trous qui définissent une pluralité correspondante de trajectoires de trous de grille et dans lequel le modèle de grille est conçu pour supporter et guider au moins un outil d'intervention le long de cette trajectoire de trous de grille lorsqu'il est inséré à travers au moins l'un desdits trous dans le corps au cours de ladite intervention, le dispositif de traitement comprenant :
- une unité de traitement d'informations spatiales anatomiques (2) pour recevoir et/ou traiter des données représentatives d'au moins un volume spatial cible dans le corps ;
- un échantillonneur de position de grille (4) pour générer une pluralité de positions et/ou d'orientations candidates du modèle de grille par rapport au au moins un volume spatial cible dans le corps ;
- un calculateur de qualité (5) pour calculer, pour chaque position et/ou orientation candidate de la pluralité de positions et/ou d'orientations candidates, au moins une mesure de qualité représentative de l'adéquation de la position candidate du modèle de grille pour l'intervention, et
- un sélecteur de position (6) pour sélectionner une position et/ou une orientation à partir de la pluralité de positions et/ou d'orientations candidates sur la base de la au moins une mesure de qualité,
dans lequel ledit calculateur de qualité (5) est conçu pour déterminer, pour chaque position et/ou orientation candidate, une relation spatiale entre chaque trajectoire de trou de grille du modèle de grille, lorsqu'il est positionné conformément à la position et/ou à l'orientation candidate, et ledit au moins un volume cible,
dans lequel ladite au moins une mesure de qualité comprend une valeur indiquant une mesure d'efficacité de traitement de l'intervention médicale lorsqu'elle est limitée par ladite relation spatiale déterminée, **caractérisé en ce que** ladite mesure d'efficacité de traitement est représentative d'une dose de rayonnement ou d'un effet d'ablation reçu dans ledit au moins un volume spatial cible lorsqu'une ou plusieurs sources de rayonnement ou un ou plusieurs dispositifs d'ablation sont positionnés le long desdites trajectoires de trous de grille.

2. Dispositif selon la revendication 1, dans lequel ladite valeur indique en outre un chevauchement et/ou une proximité géomesure des trajectoires de trous de grille par rapport audit au moins un volume cible sur la base de ladite relation spatiale déterminée.

3. Dispositif selon la revendication 1 ou la revendication 2, dans lequel ledit calculateur de qualité (5) est conçu pour déterminer ladite relation spatiale en déterminant, pour chaque position et/ou orientation candidate, une intersection entre chaque trajectoire de trou de grille du modèle de grille, lorsqu'il est positionné conformément à la position et/ou à l'orientation candidate, avec ledit au moins un volume cible, et pour calculer la au moins une mesure de qualité, pour chaque position et/ou orientation candidate, en prenant au moins une première valeur indiquant un degré d'intersection desdites trajectoires de trous de grille pour la position et/ou l'orientation candidate avec le au moins un volume cible pris en compte.

4. Dispositif selon la revendication 3, dans lequel ladite première valeur comprend un nombre total de trajectoires croisées de trous de grille avec le au moins un volume cible, et/ou une longueur totale des segments de ligne qui correspondent auxdites intersections, et/ou une moyenne, ou autre mesure statistique de tendance centrale, de la longueur desdits segments de ligne qui correspondent auxdites intersections.

5. Dispositif selon la revendication 3 ou la revendication 4, dans lequel ladite unité de traitement d'informations spatiales anatomiques (2) est en outre conçue pour recevoir et/ou traiter des données représentatives d'au moins un volume spatial à risque dans le corps,
dans lequel ledit calculateur de qualité (5) est conçu pour déterminer, pour chaque position et/ou orientation candidate, une intersection de chaque trajectoire de trou de grille du modèle de grille, lorsqu'il est positionné conformément à la position et/ou l'orientation candidate, avec ledit au moins un volume à risque,
dans lequel ledit calculateur de qualité est conçu pour calculer la au moins une mesure de qualité, pour chaque position et/ou orientation candidate, en prenant au moins ladite première valeur et une deuxième valeur, indiquant un degré d'intersection desdites trajectoires de trous de grille pour la position et/ou l'orientation candidate avec le au moins un volume à risque, pris en compte.

6. Dispositif selon la revendication 5, dans lequel ladite deuxième valeur comprend un nombre total de trajectoires croisées de trous de grille avec le au moins un volume à risque, et/ou une longueur totale des segments de ligne qui correspondent auxdites intersections avec le au moins un volume à risque, et/ou une moyenne, ou une autre mesure statistique de tendance centrale, de la longueur desdits segments de ligne qui correspondent auxdites intersections avec le au moins un volume à risque.

7. Dispositif selon l'une quelconque des revendications 3 à 6, dans lequel ledit calculateur de qualité (5) est conçu pour calculer la au moins une mesure de qualité, pour chaque position et/ou orientation candidate, en prenant en compte au moins ladite première valeur et une troisième valeur, dans lequel ladite troisième valeur indique une distance minimale d'une trajectoire de trou de grille au centre du, ou d'au moins l'un des, au moins un volume cible.

8. Dispositif selon l'une quelconque des revendications 3 à 7, dans lequel ladite au moins une mesure de qualité est une pluralité de mesures de qualité, dans lequel ledit calculateur de qualité (5) est conçu pour combiner ladite pluralité de mesures de qualité en une mesure de qualité composite conformément à une somme pondérée, et dans lequel ledit sélecteur de position (6) est conçu pour sélectionner une position et/ou une orientation à partir de la pluralité de positions et/ou d'orientations candidates pour lesquelles un extremum est atteint de la mesure de qualité composite.

9. Dispositif selon l'une quelconque des revendications précédentes, dans lequel ladite au moins une mesure de qualité est une pluralité de mesures de qualité, dans lequel ledit sélecteur de position (6) est conçu pour sélectionner un premier sous-ensemble de ladite pluralité de positions et/ou d'orientations candidates sur la base d'une première mesure de qualité de ladite pluralité de mesures de qualité, et pour sélectionner au moins un second sous-ensemble dudit premier sous-ensemble sur la base d'une seconde mesure de qualité, différente de ladite première mesure de qualité, de ladite pluralité de mesures de qualité.

10. Dispositif selon l'une quelconque des revendications précédentes, dans lequel ledit échantillonneur de position de grille (4) est conçu pour générer la pluralité de positions et/ou orientations candidates par translation et/ou rotation d'une représentation de position et/ou d'orientation du modèle de grille au cours d'une pluralité d'étapes de translation et/ou rotation différentes.

11. Dispositif selon l'une quelconque des revendications précédentes, comprenant un évaluateur d'alignement de modèle de grille (9) pour recevoir un signal de position indiquant une position et/ou une orientation physique d'un modèle de grille physique et pour fournir un signal de rétroaction indiquant la position et/ou l'orientation sélectionnée par le sélecteur de position (6) et/ou la position et/ou l'orientation physique d'une position et/ou d'une orientation relative entre ladite position et/ou ladite orientation physique et ladite position et/ou ladite orientation sélectionnée.

12. Dispositif selon la revendication 11, dans lequel ledit évaluateur d'alignement de modèle de grille (9) est conçu pour visualiser simultanément, à l'aide d'une interface utilisateur (7), la position et/ou l'orientation physique et la position et/ou l'orientation sélectionnée.

13. Procédé mis en œuvre par ordinateur (100) pour déterminer une position et/ou une orientation d'un modèle de grille par rapport à un corps humain ou animal au cours d'une intervention médicale, dans lequel ledit modèle de grille comprend une pluralité de trous qui définissent une pluralité correspondante de trajectoires de trous de grille et dans lequel le modèle de grille est conçu pour supporter et guider au moins un outil d'intervention le long de cette trajectoire de trous de grille lorsqu'il est inséré à travers au moins l'un desdits trous dans le corps au cours de ladite intervention, le procédé comprenant :
- la réception et/ou le traitement (101) de données représentatives d'au moins un volume spatial cible dans le corps,
- la génération (102) d'une pluralité de positions et/ou d'orientations candidates du modèle de grille par rapport au au moins un volume spatial cible dans le corps,
- la détermination (103) pour chaque position et/ou orientation candidate, d'une relation spatiale entre chaque trajectoire de trou de grille du modèle de grille, lorsqu'il est positionné conformément à la position et/ou l'orientation candidate, et ledit au moins un volume cible,
- le calcul (104), pour chaque position et/ou orientation candidate de la pluralité de positions et/ou d'orientations candidates, d'au moins une mesure de qualité représentative de l'adéquation de la position candidate du modèle de grille pour l'intervention en prenant en compte, au moins, une valeur indiquant une mesure d'efficacité de traitement de la procédure d'intervention médicale lorsqu'elle est limitée par ladite relation spatiale prise en compte, ladite mesure d'efficacité de traitement étant représentative d'une dose de rayonnement ou d'un effet d'ablation reçu dans ledit au moins un volume spatial cible lorsqu'une ou plusieurs sources de rayonnement ou un ou plusieurs dispositifs d'ablation sont positionnés le long desdites trajectoires de trous de grille, et
- la sélection (105) d'une position et/ou d'une orientation à partir de la pluralité de positions et/ou d'orientations candidates sur la base de ladite au moins une mesure de qualité.

14. Produit de programme informatique pour mettre en œuvre le procédé mis en œuvre par ordinateur (100) conformément à la revendication 13, lors de l'exécution du produit de programme informatique sur un processeur.
